# EUROPEAN PATENT SPECIFICATION

(11) **EP 4 323 027 B1**
(45) Date of publication and mention of the grant of the patent: **12.11.2025**
(21) Application number: 21716253.6
(22) Date of filing: 14.04.2021
(51) Int. Cl.: A61L 26/00

(54) **FLOWABLE HEMOSTATIC PASTE**
FLIESSFÄHIGE HÄMOSTATISCHE PASTE
PÂTE HÉMOSTATIQUE FLUIDE

(43) Date of publication of application: 21.02.2024
(73) Proprietor: Guangzhou Bioseal Biotech Co., Ltd., Guangzhou, Guangdong 510530 (CN); Ethicon, Inc., Raritan, NJ 08869 (US)
(72) Inventor: CHEN, Shuang, Guangzhou, Guangdong 510530 (CN); XIE, Tingwan, Guangzhou, Guangdong 510530 (CN); WANG, Yalin, Guangzhou, Guangdong 510530 (CN); LI, Yufu, Raritan, New Jersey 08869 (US)
(74) Representative: Carpmaels & Ransford LLP
(86) International application number: PCT/CN2021/087167
(87) International publication number: WO 2022/217492

(56) References cited:
- EP-A1- 1 559 438
- WO-A1-2019/160717
- WO-A2-2020/035848
- US-A1- 2020 139 021

## Description

### FIELD OF THE INVENTION

The present invention relates, *inter alia,* to flowable hemostatic compositions comprising a blend of fibrinogen, thrombin, and a dispersant.

### BACKGROUND OF THE INVENTION

In various situations during surgery therapeutic agents are spread inside the human body, for prevention of bleeding after the surgery. Existing agents have drawbacks, for example, sheets are difficult to place in minimal invasive surgeries (MIS).

The selection of appropriate methods or products for the control of bleeding is dependent upon many factors, which include but are not limited to bleeding severity; anatomical location of the source and the proximity of adjacent critical structures, whether the bleeding is from a discrete source or from a broader surface area; visibility and precise identification of the source; and access to the source.

In an effort to address the above-described problems, materials have been developed for controlling excessive bleeding. Topical Absorbable Hemostats (TAHs) are widely used in surgical applications. TAHs encompass products based on oxidized cellulose (OC), gelatin, collagen, chitin, chitosan, etc. To improve the hemostatic performance, scaffolds based on the above materials can be combined with biologically-derived clotting factors, such as thrombin and fibrinogen.

Due to its biodegradability, and its bactericidal and hemostatic properties, oxidized cellulose (OC) based materials such as oxidized regenerated cellulose (ORC), have long been used as topical hemostats. OC- and ORC- based materials are also used as an adhesion barrier. Products based on ORC are used in a variety of surgical procedures, including: neurosurgery, abdominal surgery, cardiovascular surgery, thoracic surgery, head and neck surgery, pelvic surgery and skin and subcutaneous tissue procedures. Several methods for forming various types of hemostats based on OC materials are known, whether made in powder, woven, non-woven, knit, and other forms. Currently utilized hemostats include powder, or fabrics comprising ORC.

However, since control of bleeding is essential and critical in surgical procedures to minimize blood loss, to reduce post-surgical complications, and to shorten the duration of the surgery in the operating room, there is a need for improved forms and materials which facilitate ease of application, especially in bleeding sites and in hard-to-reach areas.

US Patent Application 2020/0,139,021 discloses compositions comprised of oxidized cellulose (OC) and glycerol, with the ratio of glycerol to OC being at least about 0.5:1 w/w glycerol:OC and/or with the viscosity of the composition being at least 10% higher than that of the glycerol and lower than about 2.6×10⁹ mPa·s (cP) with the total water content being less than about 8% w/w.

US Patent No. 9,265,858 discloses a dry composition, which upon addition of an aqueous medium forms a substantially homogenous paste suitable for use in haemostasis procedures.

WO2019/160717 describes hemostatic compositions comprising at least partially integrated agglomerated ORC fibers, fibrinogen, and thrombin and methods of forming a powdered hemostatic composition, comprising the steps of: forming a suspension of a mixture comprising particles of fibrinogen, thrombin, ORC fibers in a non-aqueous low boiling solvent, agitating and shearing said suspension in a high shear mixing reactor, adding water to allow particles to agglomerate, allowing the non-aqueous solvent to evaporate, drying and sieving the composition; and thus forming the powdered hemostatic composition.

EP1559438 describes sterilized and unsterilized hemostatic compositions that contain a biocompatible liquid having particles of a biocompatible polymer suitable for use in hemostasis and which is substantially insoluble in the liquid, up to about 20 percent by weight of glycerol and about 1 percent by weight of benzalkonium chloride, each based on the weight of the liquid, all of which are substantially homogenously dispersed throughout the liquid to form a substantially homogenous composition, methods for making such compositions, medical devices that contain such hemostatic compositions disposed therein and methods of making such devices.

WO2020035848 describes methods for large scale preparation of sterile stable liquid thrombin composition comprised of glycerol; stable liquid thrombin compositions; and hemostatic composition and kits.

### SUMMARY OF THE INVENTION

The present invention relates, *inter alia,* to flowable hemostatic compositions comprising a blend of fibrinogen, thrombin, and a dispersant.

An object of the present invention is to provide a composition for preparing a pasty spreadable hemostat having a therapeutic or medicinal value, which may easily be applied to a site of need, including in difficult-to-reach areas of the body.

Fibrinogen interacts with thrombin in aqueous media. Finding a suitable formulation having an appropriate consistency is not straight-forward, since the commonly used dispersant, such as water or hydrophilic dispersant, cannot be used without compromising the pastiness, adhesiveness, and/or hemostatic functions. Thus, for a stable liquid/flowable composition comprising both fibrinogen and thrombin, non-aqueous medium (dispersant) is commonly used prior to application on bleeding sites e.g., for sealing and hemostasis. Finding a suitable formulation to bring the OC to an appropriate consistency is also not straight-forward, since the commonly used solvates or non-solvates, such as water or oils, cannot be used without compromising the active functions of the OC.

The present inventors have developed a composition comprising e.g., oxidized cellulose (OC), for preparing a spreadable paste or powder for use as a hemostat, which may easily be applied to a site of need.

Accordingly, the present invention is based on the surprising finding that such a pasty suspension comprising a powder of OC, fibrinogen, thrombin dispersed in a dispersant comprising e.g., glycerol and aqueous solution provides superior hemostatic functionalities. Such a composition may therefore be applied to a site of need in order to obtain biological activity such as controlling bleeding especially in difficult-to-reach areas of the body.

The use of aqueous solution in such a suspension is counter-intuitive, and it may be assumed, without being bound by any particular theory or mechanism, that after mixing the powder in the dispersant the uniformly dispersed biologics forms a fibrin in a glycerol/saline system which together with the OC forms a porous paste with rich thrombin remain in the system. Thereafter, upon application in a bleeding site the porous flowable ORC-fibrin matrix (which is insoluble in water) allows blood to easily percolate through its space and to be exposed to high concentration of thrombin, thereby accelerating the formation of endogenous fibrin clot upon contact with blood. Thus, it may be assumed, without being bound to a theory, that such a porous matrix of OC-fibrin also provides an environment for red cells and platelets to adhere and agglomerate, and that the flowable matrix can flow into deep lacunas and irregular sites to stick closely to bleeding sites after being tenderly pressed.

As such, the disclosed composition has been demonstrated to be an easy-to-use, stable and efficacious hemostat.

According to an aspect of the present disclosure, there is provided a two-component flowable composition comprising: (i) a powder comprising oxidized cellulose, fibrinogen and thrombin, having a weight ratio of oxidized cellulose to fibrinogen ranging from 1:5 to 2:1, respectively; and (ii) a dispersant comprising glycerol and an aqueous solution, the composition being in the form of a pasty suspension at a room temperature.

In some embodiments, the weight ratio of the OC to fibrinogen ranges from 1:2 to 2:1.

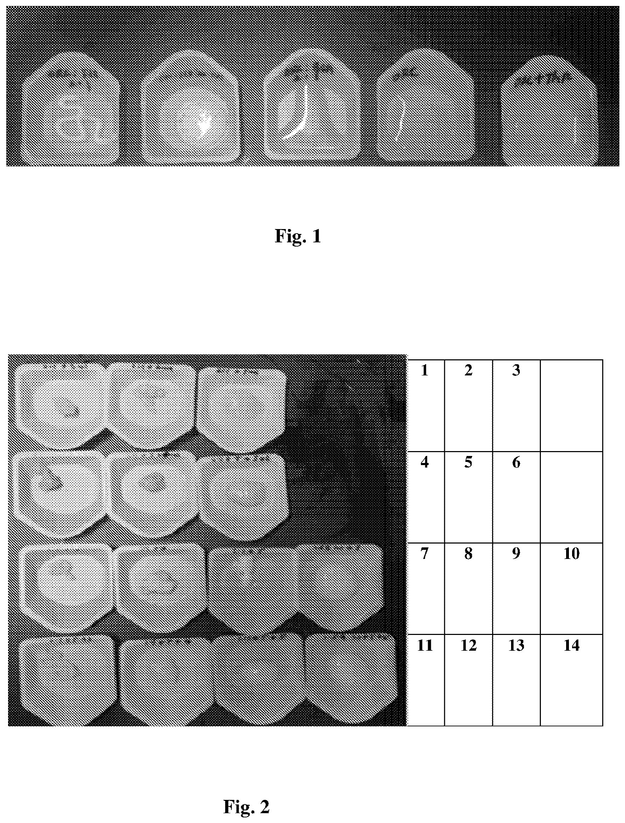

Reference is made to **Figure 2****,** showing that 3 ml dispersant (an aqueous solution comprising 50 % glycerol, by volume) per 1.2. g powder provided too is thick composition, and upon using 5 ml the composition becomes very thin. Accordingly, in some embodiments, the dispersant is present at a dispersant-to-powder ratio ranging from about 2.5:1 to about 5:1 (v/w in ml/g), respectively, e.g., 2.5:1, 3:1, 3.5:1, 4:1, or 5:1, including any value and range therebetween.

In some embodiments, the OC comprises oxidized regenerated cellulose (ORC).

In some embodiments, at least part of the powder is in an aggregated form. In some embodiments, the OC is in the milled form.

In some embodiments, the composition further comprises fibrin. In some embodiments, the fibrin is at least partial cross-linked.

In some embodiments, the thrombin is present at an amount of about 2000 to about 4000 IU per 1 gr powder.

In some embodiments, the composition comprises a calcium salt.

In some embodiments, the composition comprises a buffering agent e.g., in the form of a powder. In some embodiments, the buffering agent is selected from Tris, lysine, or a combination thereof.

In some embodiments, the particle size of at least 90 % of the powder ranges from 10 to 2,000 µm. In some embodiments, the particle size ranges from about 200 to about 900 µm.

In some embodiments, the glycerol is present at a concentration of above about 15 % by volume of the dispersant. In some embodiments, the glycerol is present at a concentration of above about 40 % by volume of the dispersant. In some embodiments, the glycerol is present at a concentration of up to about 70 % by volume of the dispersant.

In some embodiments, the composition is stable for at least about 20 h at pH of 4.5 to 6.5 at about room temperature.

In some embodiments, the aqueous solution comprises saline.

In some embodiments, the powder is spray dried and/or high sheared mixed.

In some embodiments, the fibrinogen is present at a concentration range of 60 % to 95 %, or 70 % to 90 %, by weight of the powder. In some embodiments, the composition is for use as a hemostat in a bleeding site of a tissue.

In an aspect there is provided a use of the composition in any embodiment thereof in a method for treating a bleeding tissue, the method comprising applying the composition onto the bleeding tissue. In some embodiments, the method comprises minimal invasive surgery (MIS) such as laparoscopy.

In another aspect there is provided a method of making the composition in any embodiment thereof, the method comprising combining OC (e.g., ORC) fibers/powder with a fibrinogen powder and thrombin powder in conditions allowing forming an aggregated form of the OC fibers/powder, fibrinogen and thrombin, and combining the aggregate with a dispersant comprising glycerol and an aqueous solution.

In some embodiments, the conditions allow at least partial conversion of fibrinogen into fibrin. In some embodiments, the conditions allow at least partial crosslinking of the fibrin. In some embodiments, the conditions are selected from one or more from (i) to (iii) as follows: (i) a temperature in the range of from 0 to 30 °C, (ii) pH in the range of from 4.5 to 6.5, and (iii) a dispersant being at a dispersant-to-powder ratio ranging from 3:1 to 4:1 (v/w ml/g), respectively.

In another aspect, there is provided a kit comprising container containing the composition of any embodiment thereof; and an applicator for applying the composition onto a tissue; and optionally instructions for use.

In another aspect, there is provided a kit comprising: a container containing a powder comprising oxidized cellulose, fibrinogen and thrombin, having a weight ratio of oxidized cellulose to fibrinogen ranging from about 1:5 to about 2:1, respectively; and a container containing a dispersant comprising glycerol and an aqueous solution, and optionally instructions for use.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawing. With specific reference now to the drawing in detail, it is stressed that the particulars shown are by way of example and for purposes of illustrative discussion of embodiments of the invention. In this regard, the description taken with the drawing makes apparent to those skilled in the art how embodiments of the invention may be practiced.
**Fig. 1** presents comparative photographic images demonstrating the flowability of selected samples (1.2 g powder in 4 ml 50 % glycerol by volume in saline) as described in Example 2; from left to right: powder having ORC-to-fibrinogen weight ratio of 2:1, respectively (with thrombin, i.e. 3300 IU dose per 1.2 g); (ii) powder having ORC-to-fibrinogen weight ratio of 2:1, respectively, without thrombin; (iii) a powder comprising ORC and BSA (bovine serum albumin) with ORC-to-BSA weight ratio of 2:1, with thrombin; (iv) ORC; (v) a powder comprising ORC and thrombin (without fibrinogen).
**Fig. 2** presents comparative photographic images demonstrating the flowability of selected samples according to Table 1 in the Examples section below (each panel with the corresponding sample number, from left to right).

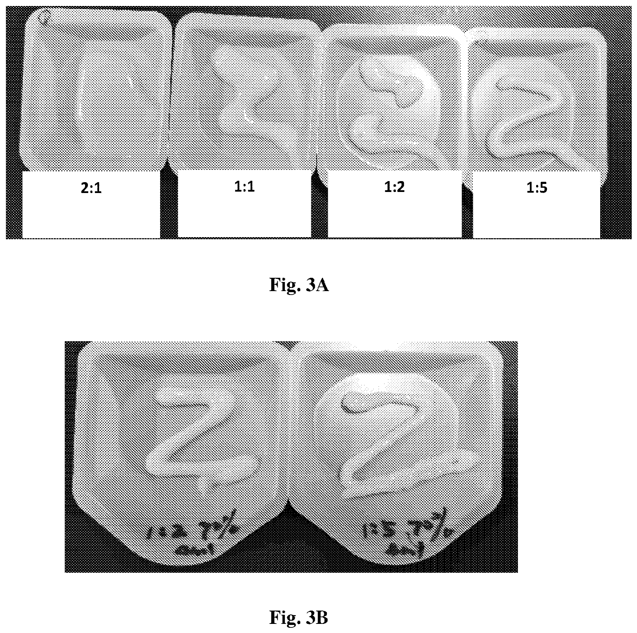

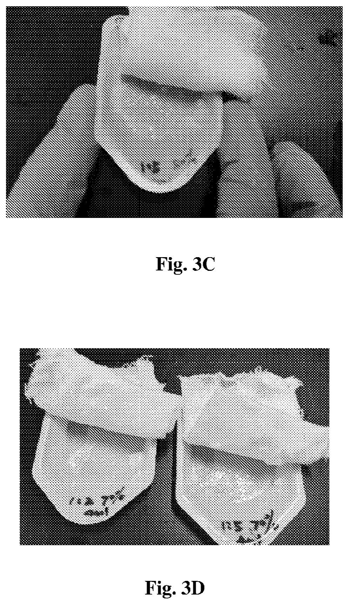

**Fig. 3A-3D** present comparative photographic images demonstrating the flowability and the respective clotting time of tested samples with various ORC-to-fibrinogen weight ratio ("ratio") ranging from 2:1 to 1:5, respectively, with 70 % or 80 % glycerol in saline solution, by volume: 80 % glycerol with ratios of 2:1, 1:1, 1:2, and 1:5 (**Fig. 3A**, from left to right); 70 % glycerol with ratios of 1:2, and 1:5 (**Fig. 3B**, from left to right); blood clotting (for the 80 % glycerol with ratios of 1:5; after above 180 sec; **Fig. 3C**); blood clotting (for the 70 % glycerol with respective ratios of 1:2 and 1:5; after 70 sec; **Fig. 3D****,** from left to right).
**Fig. 4** presents graphs showing the comparative time-dependent viscosity curves of different samples of ORC-to-fibrinogen weight ratio of 2:1 ("1"), 1:1 ("2"), 1:2 ("3"), and 1:5 ("4") dispersed in 80 % glycerol-in-saline solution, by volume.
**Fig. 5** presents graphs showing the results of amplitude sweep test, carried out on different samples of ORC-to-fibrinogen weight ratio of 2:1 ("1", "1'"), 1:1 ("2", 2'"), 1:2 ("3", "3'"), and 1:5 ("4", "4'") dispersed in 50 % glycerol-in-saline solution, by volume. In the plots: square symbols refer to G'; triangle symbols refer to G".
**Fig. 6** presents graphs demonstrating porcine blood clotting time (*ex vivo* test) with different formulations of samples after reconstitution, 2 h and 20 h later (saline and various concentration of glycerol) as described in Example 4 (see "Effect of pH and Glycerol Concentration on Stability"; circle shows solid-liquid phase separation after 20 h for sample 6).
**Fig. 7** presents graphs demonstrating porcine blood clotting time of different samples, and pH, after reconstitution, 0.5 h 2 h and 20 h later (saline and various concentrations of glycerol) as described in Example 4 (see Table 3).
**Fig. 8** presents graphs demonstrating glycerol concentration effect on thrombin stability over time with 2:1 and 1:1 ratios of ORC-to-fibrinogen formulations at various pH values (in saline + various concentration of glycerol) as described in Example 5 (see Table 4).
**Fig. 9** presents graphs demonstrating pH effect on thrombin stability over time with 2:1 and 1:1 ORC-to-fibrinogen ratios formulations at various pH values (in saline + glycerol 50 %, by volume) as described in Example 5 (see Table 5).
**Fig. 10** presents graphs demonstrating the thickness in mm of selected ORC-to-fibrinogen ratio samples (1.2 g in 4 ml 50 % glycerol by volume + saline) over time as described in Example 6 (see Table 6).
**Fig. 11** presents images showing a schematic illustration of bleeding levels grade used in the spleen biopsy punch model. The white circle represents the punch, the grey circle represents background the tissue and the black circle represents the blood flowing from the biopsy punch site (from left panel to right: No Bleeding "0"; Ooze "1"; Very Mild "2"; Mild "3"; Moderate "4"; Severe "5").
**Fig. 12** presents a bar graph summarizing the hemostasis success rate as determined in *in vivo* porcine punch model test with a 6 mm biopsy puncher using spleen biopsy punch model under heparinized condition for different formulations (N=5; see Example 7).
**Fig. 13** presents a bar graph summarizing the hemostasis success rate as determined in *in vivo* canine punch model test with a 6 mm biopsy puncher using spleen biopsy punch model under heparinized condition for different formulations (N=5; see Example 7).

### DESCRIPTION OF EMBODIMENTS OF THE INVENTION

The production of flowable hemostat of fibrin composite matrix that is easily prepared and applied for intra-operative use in difficult-to-access bleeding sites is challenging, especially during minimally invasive surgeries (MIS) such as, e.g., endoscopy.

An object of the present invention is to provide a composition which may be easily applied to a bleeding site of need, especially in difficult-to-reach areas of the body. A further advantage of the compositions of the invention is that they are bioabsorbable, and therefore may be left behind following surgery without causing any side effects.

By "applied to a bleeding site of need" it is meant to refer e.g., to a topical application of the composition at, or on/near a bleeding surgical site of a tissue.

According to an aspect of the present disclosure, there is provided a flowable composition comprising: (i) a powder comprising oxidized cellulose, fibrinogen and thrombin; and (ii) a dispersant comprising a polyol compound and an aqueous solution. In some embodiments, the composition is in the form of a suspension at around room temperature. In some embodiments, the suspension is a pasty suspension. In some embodiments, the polyol compound comprises a sugar alcohol e.g., glycerol. In some embodiments, the weight ratio of oxidized cellulose to fibrinogen ranges from about 1:5 to about 2:1, respectively. In some embodiments, the composition is a two-component composition.

In some embodiments, the composition is bioabsorbable. The term "bioabsorbable" refers to the ability of a tissue-compatible material to degrade in the body after implantation into nontoxic products which are eventually eliminated from the body or metabolized.

As used herein, and unless stated otherwise, the terms "weight ratio", "by weight", "w/w", "weight percent", or "wt. %", which are used herein interchangeably describe the concentration of a particular substance out of the total weight of the corresponding mixture, solution, suspension, formulation or composition. As used herein, and unless stated otherwise, the terms "volume ratio", "by volume", "v/v", "volume percent", or "v %", which are used herein interchangeably describe the concentration of a particular substance out of the total volume of the corresponding mixture, dispersant, solution, suspension, formulation or composition.

The two-component composition may be prepared by mixing a first component and a second component to make a suspension. As used herein, the term the "two-component composition" refers to a composition comprising at least two components. Typically, but not exclusively, the two components are stored in separate containers and are mixed to create a mixture or suspension thereof before application of the mixture or the suspension to a substrate. Specifically, in embodiments of the two-component compositions used in the present invention, the first component and the second component are mixed to make a dispersed mixture thereof within 2 hours, or, in some cases, within 0.5 hour, before application of the mixture to a substrate e.g., a bleeding site.

As used herein, the term "mixture" refers, but is not limited to, a combination of components in any physical form, e.g., blend, solution, suspension, dispersion, or the like.

The term "suspension", or any grammatical derivative thereof, as used herein, refers to a heterogeneous mixture of a solid in fine solute-like microparticles dispersed in a liquid or solvent-like phase. Typically, a suspension will have a tendency to settle, namely the fine particles of the solid matter may have the tendency to precipitate after a period of time, which, in some embodiments, is above 20 h. This period of time may depend on many factors, such as the substances of the microparticles and the liquid, the temperature, the pH, and other physical parameters like stirring and shaking, and the presence of other substances, such as dispersing agents, emulsifiers, surface-active agents, thickeners and the likes.

As used herein, the term "bleeding" refers to extravasation of blood from any component of the circulatory system. A "bleeding" thus encompasses unwanted, uncontrolled and often excessive bleeding in connection with surgery, trauma, or other forms of tissue damage, as well as unwanted bleedings in patients having bleeding disorders. The term "blood", or any grammatical inflection thereof, also includes blood fractions, such as plasma.

The term "trauma" is defined as an injury caused by a physical force; non-limiting examples include the consequences of vehicle accidents, gunshots and burns.

The term "powder" refers to solid particulate material, typically, to dispersed dry solid particles, e.g., in the form of a plurality of particles of a solid characterized by small size of at least one dimension thereof, typically, within the range of from 0.1 to 1000 micrometers, at times below 100 micrometer.

The term "solid" characterizes the state of the compound or composition at room temperature (e.g., 25 °C) and at atmospheric pressure (760 mmHg), i.e. a compound or a composition of high consistency which retains its form during storage. This term in the present application also relates to non-fluid particles, or dissolved substance. As opposed to "liquid" compounds and compositions, the solid does not flow under its own weight. "Powdered" and "particulate" may be used interchangeably herein.

The term "paste" as used herein, relates to the consistency of the composition at at-least one temperature around the room temperature, and defines a fluid mixture of solid particles. Typically, paste has no fixed shape, and is therefore not a solid or a gas. The term "paste" according to the present disclosure may also include slurry, salve, and ointment. Slurry may functionally be regarded as a thin, oily paste. A paste according to the present disclosure may also include pores comprising an expandable gas, such as air. Accordingly, in some embodiments, the composition is a paste, or is in pasty consistency, at at-least one temperature at around the room temperature.

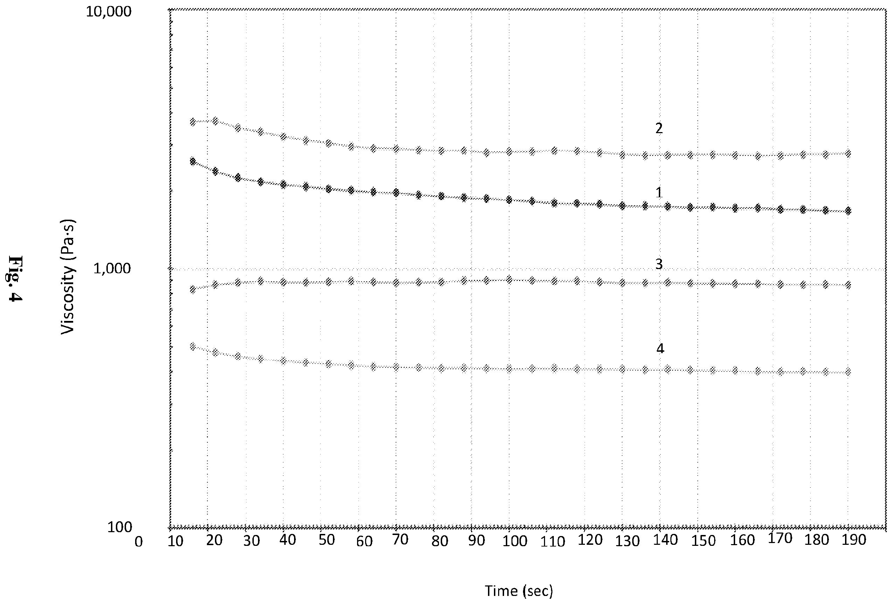

The term "flowable" also encompasses a viscous solution. The term "viscous solution" refers to a solution that has an increased resistance to flow, yet is capable of flowing. In some embodiments, the weight ratio of the oxidized cellulose to fibrinogen ranges from 1:5 to 2:1, respectively and the composition has a viscosity greater than about 400 and is less than about 3000 Pa·s. Reference is made to **Figure 4** in this regard.

In some embodiments, the paste is characterized by a viscosity of 400 to 2950, e.g., 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, or 2990 Pa·s, including any value and range therebetween.

The term "viscosity" refers to a property of fluids and slurries that indicates their resistance to flow, defined as the ratio of shear stress to shear rate. Viscosity typically has a stated or an understood shear rate. Hereinthroughout, unless stated otherwise, in the context of viscosity the shear rate is 0.5 sec⁻¹, and the viscosity is measured at ambient pressure and temperature conditions (referred to as "ambient conditions", i.e. around room temperature and atmospheric pressure). The term "shear rate" refers to the velocity gradient measured across the diameter of a fluid-flow channel, be it a pipe, annulus or other shape. Shear rate is the rate of change of velocity at which one layer of fluid passes over an adjacent layer.

In some embodiments, the composition is in an injectable form. As used herein, the phrase "injectable form" refers to a flowable composition (e.g., may comprise crosslinked fibrinogen, polymerized fibrin and/or crosslinked fibrin in the form of particles small enough to allow for injection into a human subject, e.g., injection *via* a syringe and needle), as well as being of a suitable purity and non-toxicity for injection into a subject. The composition should be homogeneous and have the rheological properties for passing smoothly while injected through needles of various internal diameters (e.g., 14 - 32 gauge). Thus, in some embodiments, the composition is capable of being passed in/through an applicator.

In some embodiments, the composition is homogeneous. As used herein, by "homogeneous" it is meant to refer to a uniform composition and texture throughout, that is, the powder is dispersed substantially evenly throughout the dispersant. Herein, "dispersed substantially evenly" is meant that the concentration of the powder within the dispersant varies within less than ± 30 %, less than ± 20 %, less than ± 10 %, less than ± 5 %, by weight, throughout the dispersant, that is, without a significant solid-liquid phase separation. Typically, term "homogeneous" refers to a uniform appearing product (e.g., paste) which withstands a liquid/solid phase separation (e.g., less than 4 % by weight) at 8 to 40 °C, 10 to 30 °C, or 20 to 30 °C.

The terms "liquid medium", "medium". or "dispersant" may be used hereinthroughout interchangeably. Herein, the term "dispersant" is meant to refer to a liquid medium, optionally a liquid medium having a viscosity having, or is capable of providing a pasty consistency. Thus, the term "dispersant" is used in a generic meaning, and may be employed rather than the word "solvent" only to clarify that the disclosed blend or powder comprised of e.g., fibrinogen, thrombin, and OC fibers or powder, is substantially not soluble in such a liquid medium, but rather forms a solid-in-liquid system.

By "around room temperature" it is meant to refer to at least one temperature value within the range of 10 °C to 40 °C, or 15 °C to 37 °C, e.g., 10 °C, 15 °C, 20 °C, 25 °C, 30 °C, 35 °C, 37 °C, or 40 °C, including any value therebetween.

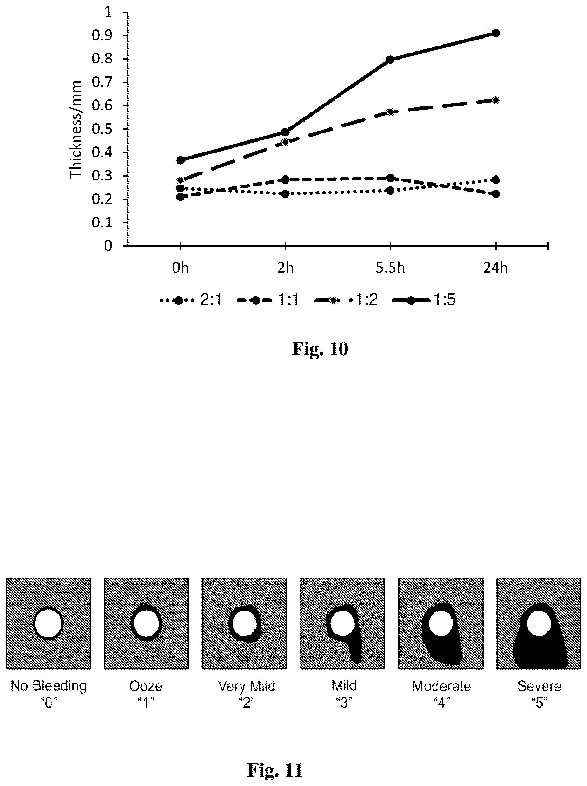

In some embodiments, the weight ratio of the OC to fibrinogen ranges from 1:5 to 2:1, respectively. In some embodiments, the weight ratio of the OC to fibrinogen ranges from 1:4 to 2:1, respectively. In some embodiments, the weight ratio of the OC to fibrinogen ranges from 1:3 to 2:1, respectively. In some embodiments, the weight ratio of the OC to fibrinogen ranges from 1:2 to 2:1, respectively. Reference is made to the Examples section, e.g., **Figure 10****,** showing that with ORC-to-fibrinogen ratio below 2:1 (1:1, 1:2, and 1:5), the reconstituted paste becomes more rigid, having a higher thickness, with at about the 1:5 ratio becoming too rigid.

The term "oxidized cellulose" (or "OC") refers to a cellulose derivative in which at least some of the primary alcohol groups, e.g., on the carbon 6 of the anhydroglucose unit is oxidized to a carboxylic acid and is optionally functionalized.

OC may be produced by applying an oxidizing agent on cellulose. The oxidizing agent may be selected from, without being limited thereto, chlorine, hydrogen peroxide, peracetic acid, chlorine dioxide, nitrogen dioxide, persulfates, permanganate, dichromate-sulfuric acid, hypochlorous acid, hypohalites, periodates, or any combination thereof, and/or a variety of metal catalysts. Oxidized cellulose may contain carboxylic acid, aldehyde, and/or ketone groups, instead of, or in addition to the original hydroxyl groups of the starting material, cellulose, depending on the nature of the oxidant and reaction conditions.

The OC used in the compositions of the present disclosure is typically, but not exclusively, in the form of a fiber or powder (also referred to as granulated OC, milled/ground OC or milled/ground OC in aggregated form). The milled/ground OC may be prepared by various methods including from existing products, and some non-limiting examples of such products are described below. As some of the existing products are in the form of a fabric, the OC powder may be prepared by grinding or milling the fabric to obtain a powder.

In some embodiments, the OC comprises oxidized regenerated cellulose (ORC).

Woven or non-woven ORC material, shredded, or ball milled ORC material may be further roller compacted to reach desired low aspect ratios and high-density ORC particles.

Examples for OC-based products that are either in the aggregated form or may be ground or milled and therefore may be utilized to prepare particles of the composition include, but are not limited to, INTERCEED^{®} absorbable adhesion barrier, SURGICEL^{®} Original absorbable hemostat, SURGICEL^{®} NU-KNIT^{®} absorbable hemostat, SURGICEL^{®} FIBRILLAR^{™} absorbable hemostat, SURGICEL^{®} SNoW^{™} absorbable hemostat and SURGICEL^{®} Powder absorbable hemostat, GelitaCel^{®} resorbable cellulose surgical dressing from Gelita Medical BV, Amsterdam, The Netherlands.

It is appreciated that while the usual source for OC is plant material, OC may also originate from a bacterial source. In some embodiments, the OC originates from a plant source.

As described herein, in one embodiment, the fibers or powder for making the disclosed composition are prepared by milling a cellulosic source material; the milling step may be preceded by forming material pieces by slitting and cutting the cellulosic source material.

In some embodiments, the disclosed composition has an OC with a carboxyl content of about 12 % to about 24 %.

As used herein with reference to OC, the terms "oxidation level", "degree of oxidation", "carboxyl content" and "carboxylation level" are interchangeable, and may be determined per United States Pharmacopeia (USP) 23-NF18.

Accordingly, in some embodiments, the carboxyl content of the OC is about 12 % - 24 % (w/w). In some embodiments, the carboxyl content of the OC is 12 % - 23 % (w/w). In some embodiments, the carboxyl content of the OC is 12 % - 22 % (w/w). In some embodiments, the carboxyl content of the OC is about 12 % to about 21 % (w/w).

Non-limiting exemplary powders comprise solid particles comprising ORC fiber and/or granules (also termed "granulated ORC").

The term "glycerol" means 1,2,3-propanetriol, and is also known as "glycerin", and may also meant to also encompass a derivative thereof. The term "derivative" refers to compound containing essential elements of the parent substance.

As used herein, "thrombin" denotes an activated enzyme which results from the proteolytic cleavage of prothrombin (factor II). Thrombin may be produced by a variety of methods of production known in the art, and includes, but is not limited to, recombinant thrombin and plasma derived thrombin.

Human thrombin is a 295 amino acid protein composed of two polypeptide chains joined by a disulfide bond. Both human and non-human (e.g., bovine) thrombin may be used within the scope of the present disclosure.

The origin for thrombin used in the present disclosure may be from one or several sources including but not limited to: plasma (e.g., porcine plasma), recombinant bacteria and/or cells (see e.g., Vu *et al.,* 2016, J. Viet. Env. 8(1):21-25), whole blood (pooled from several donations or not) and/or blood fraction (that may be pooled from several donations, e.g., plasma). Thrombin is available by manufacturers such as Johnson and Johnson, Baxter and CSL Behring either as a standalone product, e.g., EVITHROM^{®}, or as a component of a product e.g., EVICEL^{®}, TISEEL^{®}, Beriplast^{®}, and the like.

In some embodiments, the thrombin is present at an amount of about 2000 to about 4000 IU per 1 gr powder. In some embodiments, the thrombin is present at an amount of about 3000 IU per 1 gr powder. In some embodiments, the thrombin is present at an amount of about 2000, 3000, or 4000 IU per 1 gr powder, including any value and range therebetween.

Herein "U" denotes a coagulation factor unit, that is a unit of a physiological measurement of a coagulation factor in 1 ml of human normal plasma.

"IU" refers to International Unit of a coagulation factor which is a physiological measurement in a given sample as it compared to an adequate intentional standard, e.g., as determined by the clotting assay against an internal reference standard for potency concentration measurement that has been calibrated against, for example, the World Health Organization (WHO) Second International Standard for Thrombin, 01/580.

The term "fibrinogen" without more is intended to include any type of fibrinogen. "Fibrinogen" refers to monomeric and dimeric fibrinogen molecules having the monomer structure (AαBβγ), hybrid molecules, and variants thereof, whether naturally occurring, modified, or synthetic. The term "fibrinogen" may refer to fibrinogen from humans, but may include fibrinogen of any species, especially mammalian species, such as fibrinogen produced from porcine plasma.

The term "aqueous solution" refers to a solution comprising water as the solvent. This term may include water, saline, or a bleeding site. In some embodiments, the aqueous medium is selected from water, saline, a calcium chloride solution or a buffered aqueous medium.

In exemplary embodiments, the aqueous solution comprises saline. As used herein, the term "saline" refers to water containing salt at a concentration greater than about 0.5 % and less than about 20 % by weight, e.g., about 0.9 w/w % sodium chloride solution in demineralized water.

In some embodiments, at least part of the powder is in the form of aggregate(s) or granulate(s). The term "aggregate" describes a particle formed from assembled components. Aggregates may optionally be made by a step of humidifying the powder composition; compacting, e.g., by roller and/or slugging the powder to form aggregates; dehumidifying; milling; sieving the aggregates; and optionally dosing the resulting aggregates into a storage container or into a delivery device. The term "granulate" or "granulate material" may particularly denote a conglomeration of particles typically below 900 micron, below 800 micron, below 700 micron, or even below 500 micron. That is, at least part of the particles is not in the form of a discrete solid. In some embodiments, high shear mix is utilized to assemble particles of fibrinogen, thrombin, and optionally CaCl₂, and ORC into aggregates.

In another embodiment, the composition is in the form of a mixture. The mixture may comprise, in exemplary embodiments, spray-dried first microparticles that comprise fibrinogen, combined with spray-dried second microparticles that comprise thrombin, e.g., human thrombin, together forming particles of e.g., 10 microns to about or above 250 micron.

Additionally or alternatively, the powder compositions according to the present disclosure comprising the fibers and the compounds are compacted in the form of aggregates, optionally using steps of drying, milling/grounding and sieving as described in US patent 10,034,957. The sieve used defines the particle size of the powder.

In some embodiments of any aspect of the present disclosure, the plurality of particles is characterized by a median particle size of up to about 900 µm.

In some embodiments, the particle size of at least 90 % of the powder ranges from 10 to 2,000 µm. In some embodiments, the particle size of at least 90 % of the powder ranges from 10 to 1,000 µm. In some embodiments, the particle size of at least 90 % of the powder ranges from 100 to 1,000 µm. In some embodiments, the particle size of at least 90 % of the powder ranges from 200 to 1,000 µm. In some embodiments, the particle size of at least 90 % of the powder ranges from 100 to 900 µm. In some embodiments, the particle size of at least 90 % of the powder ranges from 200 to 900 µm.

In some embodiments of any aspect of the present disclosure, the plurality of particles is characterized by a median particle size of about 100 to about 900 µm. In some embodiments of any aspect of the present disclosure, the plurality of particles is characterized by a median particle size of about 200 to about 900 µm.

The term "median", also referred to as "D50" in the context of particles size refers to the particle size that divides the population in half such that 50 % of the population is greater than or less than this size.

The term "at least part of the powder is in an aggregated form" refers to a part of the population of the particles in the powder e.g., at least between 10 % and 100 % forming an aggregate.

In some embodiments, the composition further comprises fibrin.

The term "fibrin" does not only refer to fully coagulated fibrinogen but further includes any mixture of fibrin and fibrinogen which may occur during formation of fibrin from fibrinogen using thrombin and, thus, includes any ratio of fibrinogen/fibrin and any grade of gelation and/or clotting conceivable as long as it has no negative impact on the final pasty texture of the composition. Moreover, the term "fibrin" further includes any partly or fully cross-linked, gelled or clotted form.

It is thus to be appreciated that fibrin *per se* exists as a polymeric, insoluble matrix comprising fibrils, each fibril comprising many fibrin molecules.

In some embodiments, the glycerol is present at a concentration of above about 15 % by volume of the dispersant. In some embodiments, the glycerol is present at a concentration of above about 40 %, by volume of the dispersant. In some embodiments, the glycerol is present at a concentration of up to about 80 %, by volume of the dispersant. In some embodiments, the glycerol is present at a concentration of up to about 70 %, by volume of the dispersant. In some embodiments, the glycerol is present at a concentration of up to about 60 %, by volume of the dispersant.

In some embodiments, the glycerol is present at a concentration of above about 15 % to about 80 %, by volume of the dispersant. In some embodiments, the glycerol is present at a concentration of above about 15 % to about 80 %, by volume of the dispersant. In some embodiments, the glycerol is present at a concentration of above about 15 % to about 60 %, by volume of the dispersant. In some embodiments, the glycerol is present at a concentration of above about 40 % to about 80 %, by volume of the dispersant. In some embodiments, the glycerol is present at a concentration of above about 40 % to about 60 %, by volume of the dispersant.

In some embodiments, the glycerol is present at a concentration of about 15 %, about 20 %, about 25 %, about 30 %, about 35 %, about 40 %, about 45 %, about 50 %, about 55 %, about 60 %, about 65 %, about 70 %, about 75 %, or about 80 %, by volume of the dispersant, including any value and range therebetween.

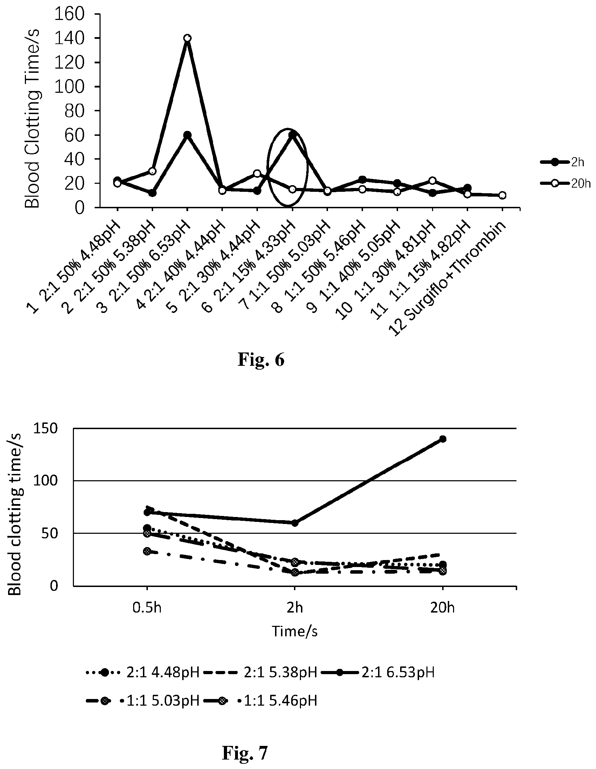

Reference is made to **Figures 3A-D**showing that the 70 % glycerol samples provided a pasty composition upon the dispersion in the above-mentioned dispersant, whereas 80 % glycerol samples exhibited higher flowability, mainly for the ORC-to-fibrinogen weight ratio ("the ratio") of 2:1 to 1:2 samples, resulted in composition being less capable of staying at a wound site, and also in a slower clotting rate (above 180 s for the 1:5 sample; the blood clotting time for the 70 % glycerol samples, 1:2 and 1:5 ratios, was 70 sec). Further results showed that (i) for the ORC-to-fibrinogen weight ratios of 2:1 to 1:1, the preferred concentration of glycerol in the dispersant (without compromising the enzyme activity, as described below) is above 15% to less than 50%, by volume, providing a clotting time of less than 60 sec for each time point; and that (ii) a liquid-solid phase separation occurred for the 15 % glycerol sample after 20 h, as shown in **Figure 6****.**

In some embodiments, the powder is spray-dried and/or high sheared mixed.

The term "spray drying" is used herein in a broad sense, which include, without being limited thereto, processes for transforming a solid dissolved or suspended in a liquid into a powdery. Typically, spray drying is a drying method used to create powder from a solution, suspension or emulsion that is atomized through a spray nozzle in a hot airflow and dried instantly. Spray drying process for producing thrombin and fibrinogen powders, which maintain their activity may be controlled by several process parameters, among them are, without limitation: column air flow and temperatures, nozzle size and atomizing air flow rate, and material flow rate. Non-limiting exemplary parameters used in order to establish a robust process to produce a blend of thrombin and fibrinogen (e.g., in a powder form) which maintains its activity are provided in the Examples section below.

The term "high-shear mixing" generally means a turbulent-flow type of mixing. In some embodiments, the high-shear mixing is characterized by speed of mixing blade and/or speed of cutting blade ranging from: 150 to 500 rpm.

Additionally, or alternatively, other methods may be used for obtaining dry composition, such as, without limitation, lyophilization of the fibrinogen solution and/or thrombin solution, followed by micronization. Typically, following lyophilization of the solution, a porous and spongy solid material, referred to as a "cake" (may also be referred to herein as "solid composition") is obtained.

The term "micronization" is used when the particles that are produced e.g., from the solid composition, are only a few micrometers in diameter. Micronization can be achieved by processes including, but not limited to, jet milling, pearl-ball milling, high-pressure homogenization, the RESS (Rapid Expansion of Supercritical Solutions) process, the SAS (Supercritical Anti-Solvent) method, or the PGSS (Particles from Gas Saturated Solutions) method. Typically, but not exclusively, micronization results in a 30 to 400- fold size reduction of the protein powder from its original size.

In some embodiments, the composition is stable.

The terms "stable", and "stability" when referring to the disclosed paste, mean that an active component within, e.g., thrombin at a certain temperature and after certain time duration remains at least 70 % active, that is, capable of forming a fibrin clot.

In some embodiments, the composition is a storage stable composition. The term "storage stable" is to be understood as referring to the formulation, composition, typically being present in a container e.g., vial, or syringe that is stable under preselected storage conditions (e.g., pH), including pre-selected storage temperature, pre-selected physical state of the formulation. Stability can be determined by methods known in the art, e.g., by testing the absence of visible aggregations, remnant and/or fibrin clots in the formulation under the preselected storage condition, for example, when the composition is in liquid or pasty form. Further, in accordance with the present disclosure, when referring to a stable sealant composition or formulation it is to be understood as one that, upon use, has an effective clotting time irrespective of some formulation's storage conditions, e.g., the sealant formulation clots at essentially the same time period irrespective of whether it was stored at room temperature (e.g., 8 to 40 °C) or at even lower temperatures (e.g., below 8 °C).

In some embodiments, the composition has pH of about 4 to about 6.5 at about room temperature. Reference is made in this regard to the Examples section below, e.g., **Figures 7-**9. In some embodiments, the composition has pH of about 4, 4.5, 5. 5.5, 6, or about 6.5, including any value and range therebetween, at about room temperature

In some embodiments, the composition in any embodiment thereof remains stable for at least about 20 h at pH of 4 to 6.5 at about room temperature. In some embodiments, the composition in any embodiment thereof remains stable for at least about 20 h at pH of 4 to 5 at about room temperature.

In some embodiments, less than 25 %, less than 24 %, less than 23 %, less than 23 %, less than 22 %, less than 21 %, or less than 20 %, by weight, remnant is detected after at least 20 h, at least 2 days, at least 3 days, at least 4 days, at least 5 days, at least 6 days, or at least 7 days at pH of 4 to 6.5 at about room temperature.

In some embodiments, the composition, in any embodiment thereof, further comprises an additional pharmaceutically active agent being contained within the composition or on a surface of the composition.

In some embodiments, the pharmaceutically active agent is selected from the group consisting of a therapeutically active agent and a labeling agent.

In some embodiments, the therapeutically active agent is selected from the group consisting of a stem cell, a growth factor, a bone morphogenetic protein, a cell, a cytokine, a hormone, a medicament, a mineral, a plasmid with therapeutic potential, and a combination thereof. As described herein, in some embodiments, the composition may further comprise at least one biologically active agent. Non-limiting biologically active agents that may be included in the composition include calcium, as well as therapeutic agents such as antibiotics, anti-inflammatory agents, growth factors, or clotting factors. Specifically, it is also possible that the disclosed composition comprises components which encourage the formation of the clot, such as, without being limited thereto, calcium salt, Factor VIII, Factor XIII, fibronectin, vitronectin, von Willebrand factor (vWF), which can be provided as a separate component or formulated with the blend or the paste.

The pharmaceutically active agent is optionally attached to the protein (i.e. fibrinogen or fibrin) matrix (e.g., covalently linked to the protein). The agent may be attached to the matrix after the matrix has been prepared and/or attached to the ingredients (e.g., protein and/or reducing sugar) which are then used to prepare the protein matrix, e.g., crosslinked fibrin.

Alternatively or additionally, the agent is absorbed by the crosslinked protein matrix. Absorption may be obtained, for example, by contacting a crosslinked fibrinogen or fibrin matrix or a non-crosslinked fibrin matrix with a solution containing the agent (e.g., by dipping, soaking, washing), or by adding the agent prior to crosslinking (e.g., inclusion of the agent in the crosslinking solution, inclusion of the agent in a solution of fibrinogen and thrombin).

Compositions described herein, as well as the contents of the abovementioned kits, may, if desired, be presented in a pack or dispenser device, which may contain one or more unit dosage forms containing the composition or ingredients (e.g., fibrinogen,) and/or reagents (e.g., dispersant) for preparing the composition. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration (FDA) for prescription drugs or of an approved product insert. Compositions comprising a preparation of the invention formulated in a pharmaceutically acceptable carrier may also be prepared, placed in an appropriate container, and labeled for use for an indicated application and/or for treatment of an indicated condition, as further detailed herein. The term "preparation" refers to a physiologically suitable for therapeutic use.

It will be appreciated that compositions of embodiments of the present disclosure may be attached to or included in medical devices, such as for promoting wound healing.

In another aspect of the present disclosure, the disclosed composition in any aspect or embodiment thereof is for use in a method for preparing a fibrin sealant in/on an injured tissue of a subject, e.g., by applying the disclosed composition in any aspect and/or embodiment thereof on a surface of the tissue.

In another aspect of the present disclosure, there is provided a method for preparing a fibrin sealant in/on an injured tissue of a subject, e.g., by applying the disclosed composition in any aspect and/or embodiment thereof as a hemostat on a surface of the tissue.

In another aspect of the present disclosure, there is provided a method of making the composition according to an embodiment thereof, the method comprising combining OC or ORC fibers with fibrinogen powder and thrombin powder in conditions allowing forming an aggregated form of the ORC fibers, fibrinogen and thrombin, and combining the aggregate with a dispersant comprising glycerol and an aqueous solution.

In some embodiments, such conditions allow at least partial conversion of fibrinogen into fibrin. In some embodiments, the conditions allow at least partial polymerization of the fibrin, e.g., by adding a certain amount of aqueous solution in the dispersant, as detailed hereinthroughout.

Conditions that allow forming a powder/aggregated form of the ORC fibers, fibrinogen and thrombin include, without being limited thereto, micronization of powders and OC fibers, e.g., by high shear process, the pH of the dispersant, e.g., 4 to 6, and temperature ranges such as, without limitation, 10 to 50 °C, 45 to 50 °C, or 20 to 30 °C.

In the context of the present invention, the term "sealant", also referred to as "fibrin sealant", and "biological glue", is to be understood as an adhesive, glue, or hemostat, e.g., such as one originates or being derived from the disclosed composition, having ingredients that upon contact with, or in proximity to, a tissue and/or blood, reacts to subsequently form a clot, may further act as a tissue adhesive, and thereby prevents, reduces, or stops bleeding, joins structures and/or seals physiological leaks, e.g., of cerebrospinal fluids (CSF), lymph, bile, gastrointestinal (GI) content, air leak from lungs etc. In some embodiments, the sealant formulation also comprises one or more therapeutics such that upon natural degradation of the clot formed in the body, the therapeutic is released. The therapeutic may be, without being limited thereto, drug(s), such as antibiotics, analgesics, anti-inflammatory drugs, cancer drugs etc., cells including, for example, any type of stem cells e.g., Embryonic Stem (ES) cells, adult stem cells, Induced Pluripotent Stem Cells (iPSCs) etc. from human or other origin.

The term "hemostatic" refers to an ability to prevent, reduce, or stop blood loss e.g., from wounds, such as surgical or traumatic wounds, e.g., by promoting blood clot formation.

Hemostasis" (or "haemostasis") refers to the first stage of wound healing. It is a process which causes bleeding to stop. By "assist in hemostasis" it is meant to help reduce or stop bleeding. By "applied to a bleeding tissue" it is meant to refer to e.g., a topical application of the composition at the bleeding site, e.g., at a surgical site to control bleeding. Control of bleeding is needed in various situations including treatment of wounds.

As used herein, the terms "controlling", "preventing", or "reducing", which may be used herein interchangeably in the context of the bleeding, including any grammatical inflection thereof, indicate that the rate of the blood extravagated is essentially nullified or is reduced e.g., by 10 %, at least 20 %, at least 30 %, at least 40 %, at least 50 %, at least 60 %, at least 70 %, at least 80 %, at least 90 %, or even by 100 %, of the initial rate of bleeding, compared to situation lacking the contact of the disclosed composition in/on the bleeding site. Methods for determining a level of appearance of bleeding are known in the art.

Further, in some embodiments, the terms "controlling", "preventing" or "reducing", in the context of the bleeding are also meant to encompass at least partially sealing blood vessels at the bleeding site e.g., in soft tissues.

As used herein, the term "subject" shall mean any animal including, without limitation, a human, a mouse, a rat, a rabbit, a non-human primate, or any other mammal. In some embodiments, the subject is human, e.g., a human patient. The subject may be male or female.

As used herein, the term "injured tissue", (or "damaged tissue") refers to disruption of the normal continuity of structures caused by a physical (e.g., mechanical) force such as in incisions caused by surgery, a biological (e.g., thermic or actinic force), or a chemical means. The term "injured tissue" also encompasses contused tissues, as well as incised, stabbed, or lacerated tissue, as well as open, puncture, and injuries caused e.g., by ripping, scratching, pressure, and biting. The term "injured tissue" also encompasses wounds and bleeding sites as described hereinthroughout.

The term "tissue" may refer to a tissue selected from a soft tissue and a bone tissue.

The term "soft tissues" as used herein relates to body tissue that is not hardened or calcified. This term especially relates to soft tissues that are vascularized and therefore may be a source of bleeding. Examples for such tissues include but are not limited to connective tissue (such as tendons, ligaments, fascia, skin, fibrous tissues, fat, and synovial membranes), muscles, and internal organs. In general, soft tissues are meant to exclude bone tissue.

Thus, in some embodiments, the disclosed composition in any embodiment thereof may be used applied in difficult-to-reach bleeding sites, for example during minimally invasive surgeries (MIS) such as, e.g., endoscopy. One of the common forms of endoscopy is laparoscopy which is minimally invasive inspection and surgery inside the abdominal cavities.

The term "surgery" also encompasses the time during or after surgical or diagnostical procedures. Further non-limiting examples of procedures include neurosurgery, abdominal surgery, cardiovascular surgery, thoracic surgery, head and neck surgery, pelvic surgery and skin and subcutaneous tissue procedures. For at least one of these situations, the composition of the invention may serve as a suitable sealant, which may permit adhesion to the bleeding wound, and thus addresses hemostasis without being dependent on the condition of the wounded tissue, e.g., severity of bleeding. The formation of the sealant occurs in a relatively short period of clotting time subsequent applying the disclosed composition, in any aspect and/or embodiment thereof, on an injured tissue. Typically, using after 1 to 3 min tamponade, an incomplete clot may form which enables to stop bleeding, but more time may be needed for clotting reacting up to the paste surface thus forming a complete clot.

In another aspect of the present invention, there is provided a composition for use in a method of treating a wound comprising the step of applying (e.g., contacting) the disclosed composition in any aspect and/or embodiment thereof onto and/or into the wound of a subject in a need thereof.

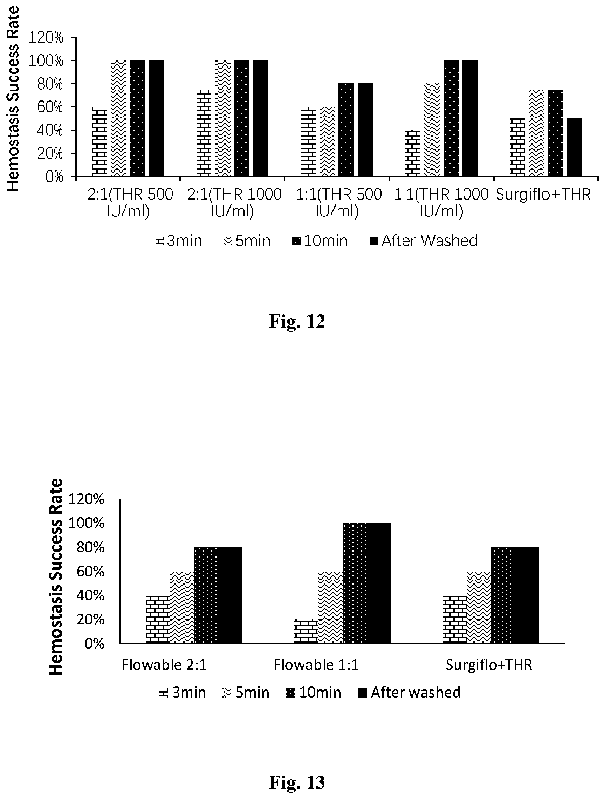

By "treating a wound" it is further meant to encompass reducing blood loss at a bleeding site of a tissue (*in vivo*), e.g., in a patient undergoing surgery. Reference is made to **Figures 12** and **13****,** showing a high hemostatic success rate (above 60 %) of the tested samples disclosed herein.

Accordingly, in some embodiments, the method is for reducing blood loss at a bleeding site of a tissue and/or making a sealant layer in/on such a tissue, e.g., in a patient undergoing surgery, the method comprising contacting the disclosed composition in an embodiment thereof with the bleeding site and/or its proximity. Optionally, the method comprises first applying on such a tissue an aqueous solution, such as saline, and thereafter applying on the aqueous solution the disclosed composition, so as to promote a fast clotting-time. Accordingly, in some such embodiments, the composition, in any embodiment thereof is for use as a hemostat in a bleeding site of a tissue.

According to another aspect of embodiments of the present invention, a kit is provided for generating a composition described herein. It is appreciated that the consistency of the composition is such that it can be applied, for example, by spreading or by sticking the composition directly onto the bleeding site. Accordingly, the composition does not need to be further spread on or applied to a solid surface, object, or other solid medium such as a strip or a film in order to be in the appropriate form for applying onto the bleeding site. Nevertheless, a suitable applicator, such as, for example, a syringe, may be used in order to apply, spread or stick the composition onto the bleeding site, for the purpose of easy access and handling.

In an additional aspect and/or embodiment, the present invention provides a kit comprising: a) a container containing the composition of the invention as described above, b) an applicator for applying the composition to a tissue, and c) optionally instructions for use.

In another embodiment, the present invention further provides a hemostatic kit comprising a container containing the herein disclosed composition in an embodiment thereof.

According to some embodiments, there is provided a kit for preparing a composition comprising (i) a powder comprising oxidized cellulose, fibrinogen and thrombin, having a weight ratio of oxidized cellulose to fibrinogen ranging from 1:5 to 2:1, respectively; and optionally (ii) a dispersant comprising glycerol and optionally an aqueous solution, the composition being in the form of a pasty suspension at a room temperature. Each of (i) and (ii) may be present in a separate container.

Any aspect and embodiment of the hereinthroughout disclosed composition may be incorporated to the aspect and embodiments of the kit, including embodiments of the composition, the dispersant, and/or the powder.

Optionally, the fibrinogen and thrombin are each packaged individually (e.g., in dry form or in solution) in the kit. Thus, each of the two ingredients is packaged in separate packaging material, in addition to the packaging material of the whole kit.

Alternatively, the fibrinogen, thrombin and oxidized cellulose is packaged together (e.g., as a dry powder) in the kit in the same packaging material, and optionally the dispersant is provided in an additional container. In some embodiments, the kit further comprises instructions on how to combine the ingredients of the kit and/or how to combine the ingredients of the kit with an additional ingredient (e.g., a dispersant), in order to produce the desired composition.

Optionally, the kit further comprises a dispersant. Dispersant such as glycerol may be in a pure form or in a solution with another liquid (e.g., water, saline or aqueous buffer). Optionally, the dispersant is packaged individually, apart from the powder. Alternatively, the dispersant is packaged in combination with the powder, for example, as a ready-for-use composition described herein. In such embodiments, the kit may further contain a measuring means, e.g., a measuring cylinder, to measure the volume of the dispersant or a component thereof.

Additionally or alternatively, the hemostatic kit may comprise a syringe containing the blend, mixture or powder and another syringe containing the dispersant. For example, dual-syringe mixing devices may produce a substantially homogenous paste mixture by combining initially separate liquid and powders and then passing the blended contents back and forth between two connected syringes *via* interconnected outlet(s). Therefore, a low expression force for dispensing the paste from a syringe may be preferred for ease of mixing and ultimately for deployment of the resulting paste. The desired expression force may be less than 1.51 lbf.

In some embodiments, at least one of the containers in the kit is a pre-filled syringe. In some embodiments, a syringe is provided in addition to the container(s) of the kit. In some embodiments, the container is in a specific type, such as a vial or an applicator such as syringe.

In some embodiments, at least one of the containers in the kit is a pre-filled syringe. In some embodiments, a syringe is provided in addition to the container(s) of the kit. The term "container" may refer to any generic structure such as a vessel or a vial, that may contain the paste.

The kit may be applied using an applicator device which may be used for administering several and sequential injections of the composition. In one embodiment, the applicator device enables multiple injections of a fixed-dose of the mixed components on a 2-D surface of a tissue while moving the device. In one embodiment, the applicator has a syringe with an injection needle, which is optionally automatically retracted from the patient's skin after the injection is completed without the need for the administrator to lift the device upward from the injection surface. In one embodiment, the kit may be used for the administration of a sealant.

The hemostatic kit of the invention may be a kit for use in reducing, preventing or stopping blood flow, e.g., in open wounds, and it may be used for reducing, preventing or stopping blood flow during a procedure, such as during, before, or after a surgical procedure such as, for example, laparoscopic surgery, neurosurgery, abdominal surgery, cardiovascular surgery, thoracic surgery, head and neck surgery, pelvic surgery and skin and subcutaneous tissue procedures. The kit may be used for reducing or preventing blood flow from the skin, or in internal organs.

In one embodiment, this kit may be stored at room temperature, such as at a temperature in the range of 8 to 40 °C, or at lower temperatures.

In some embodiments of any aspect of the kit or the composition disclosed herein, the powder or blend may comprise an additive e.g., calcium salt and/or one or more excipients, e.g., selected from, without being limited thereto, one or more amino acids, albumin, saccharides, and/or saccharide derivatives.

The term "additive" is meant to be understood as any substance that can be added to a composition, and may also include an active additive such as calcium salt as described below.

The term "excipient" as used herein denotes a non-active or non-therapeutic agent added to a pharmaceutical composition e.g., to provide a desired consistency or stabilizing effect.

Calcium is an important element in the clotting cascade, and may be needed for activation of factor XIII into factor XIIIa, which cross-links and stabilizes fibrin to generate an insoluble clot.

Accordingly, in some embodiments of any aspect of the disclosed kit and/or composition, the blend, mixture or powder further comprises an additive such as, without limitation, calcium. Calcium used with the invention may be in the form of a salt, e.g., calcium chloride salt. Alternatively, additional salts may be used, such as calcium acetate and/or calcium citrate. In the kit, the calcium salt may be provided in the composition comprising the powder. Alternatively, the excipient(s), and/or the calcium salt, may be provided in the kit in a separate container, or the excipient(s), and/or the calcium salt, may be provided in the kit in the same container comprising the blend/mixture/powder component.

In some embodiments of the kit or the composition, the calcium salt, e.g., calcium chloride (CaCl₂) is present at a concentration of 0.5 % to about 4 %, or 2 % to 4 %, or 2.5% to 3.5 %, by weight of the powder. In some embodiments, the calcium salt, e.g., calcium chloride is present at a concentration of 0.5 %, 1 %, 1.5 %, 2 %, 2.5 %, 3 %, 3.5 %, or 4 %, by weight of the blend, including any value and range therebetween.

It is known that OC is acidic and this acidity causes plasma proteins such as thrombin and fibrinogen to denature immediately; therefore, the co-administration of oxidized cellulose and thrombin and/or fibrinogen may lead to the plasma proteins being rendered ineffective.

In some embodiments, a buffering agent is added to the composition for pH adjustment.

In some such embodiments, a buffering agent e.g., Tris, or Tris(hydroxymethyl)aminomethane buffer, or lysin is added in a powder form to the fibrinogen, thrombin, and ORC blend/mixture/powder for pH adjustment (e.g., to pH of about 7). In some embodiments, the buffering agent e.g., Tris or lysine, is present at a concentration of, 0 to 25 %, 4 % to 8 %, or 5 % to 7 %, by weight of the blend/mixture/powder. In some embodiments, the buffering agent e.g., Tris or lysine, is present at a concentration of 1, 2, 3, 4, 5, 6, 7, 8, 9, 10, 11, 12, 13, 14, 15, 16, 17, 18, 19, 20, 21, 22, 23, 24, or about 25 %, by weight, including any value and range therebetween.

In some embodiments of any aspect of the kit and/or compositions, the powder further comprises an excipient selected from amino acids e.g., lysine. In some embodiments of any aspect of the kit and/or compositions, the blend or powder comprises OC and lysine.

In some embodiments, the fibrinogen is present at a concentration range of 20 % to 80 %, 60 % to 95 %, or 70 % to 90 %, by weight of the powder. In some embodiments, the fibrinogen is present at a concentration of 20 %, 30 %, 40 %, 50 %, 60 %, 70 %, 80 %, or 90 %, by weight of the powder, including any value and range therebetween.

In exemplary embodiments, the blend or the powder comprises 20-80 % fibrinogen powder, by total weight of the powder, by weight; 2000-4000 IU thrombin/dose powder; 10-50 % ORC powder, by total weight of the powder; 2-4 % CaCl₂ powder, by total weight of the powder; 0-25 % lysine powder, by total weight of the powder. Herein "dose" means about 1.2 g.

In some embodiments of any aspect of the kit and/or compositions, the disclosed composition may be used in conjunction with a backing, pad, bandage, gauze, sponge, scaffold, or matrix to provide mechanical strength to cover the wound surface. In this case, the instant matrix is supported on a pad for ease of application or tamponade.

In some embodiments of any aspect of the kit and/or compositions, the composition is sterile. Especially when handling blood products, the sterility issue is crucial, and specifically the issue of viral inactivation. In general, viral inactivation may be carried out by any number of methods, including solvent detergent, heat inactivation, irradiation, and nanofiltration. Typically, the standard for viral inactivation requires using two different methods. Additionally, FDA standard for sterility requires filtration.

The term "sterile" as used herein means having a low bioburden, effectively being germ-free, e.g., essentially or even absolutely being free from microorganisms, e.g., bacteria and viruses. Sterilization is the process of reducing the bioburden to an effectively germ-free level. A sterile liquid or paste is generally defined as a liquid or paste that has underwent sterile filtration.

The terms "comprises", "comprising". "includes", "including", "having", and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In those instances where a convention analogous to "at least one of A, B, and C, etc." is used, in general such a construction is intended in the sense one having skill in the art would understand the convention (e.g., "a composition having at least one of A, B, and C" would include but not be limited to compositions that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B".

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

The term "about" as used herein means that values that are 10 % above or below the indicated value are also intended to be included. Unless otherwise indicated, all numbers such as those expressing, for example, ratios, weight, mole/mole, amounts, viscosity, temperatures, etc., are to be understood as being modified in all instances by the term "about". Accordingly, unless indicated to the contrary, the numerical parameters set forth in this description and attached claims are approximations that may vary by up to plus or minus 10 % depending upon the desired properties sought to be obtained by the present invention.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### EXAMPLE 1: THE PRODUCTION PROCESSES OF ORC, THROMBIN, AND FIBRINOGEN POWDERS

### Materials and Methods:

The source of fibrinogen and thrombin was porcine blood plasma which was fractionated to obtain fibrinogen and thrombin, and supplied by Bioseal Biotech CO. LTD, Located in Guangzhou, China;
Bovine serum albumin (BSA)- SIGMA VETEC, V900933;
L-lysine- ALADDIN, L103479-500 g;
Oxidized regenerated cellulose (ORC): ORC characteristic: particle size < 70 µm, fiber form. The ORC powder was obtained by processing of the SURGICEL^{®} original fabric. Briefly, the ORC powder was obtained by processing of the SURGICEL^{®} original fabric in the following steps: 1) splitting and cutting the fabric into about 2" x 8" pieces; 2) milling the fabrics into the powder particle size (typically, but not exclusively, D50 less than 94 microns) using known milling methods; placing about 100 grams of fabric into a 500-ml zirconia jar, then placing 12 to 13 pieces of 20 mm zirconia balls (agates) into the same jar, covering and fix the jar in a Retsch planetary ball mill (Model PM100), milling the fabric with 450 rpm for 20 minutes, transferring the milled powders onto a 8" diameter and 300 - micron opening sieve, separating the agates and the powders by slightly shaking, and finally collecting the powders.

In exemplary procedures, the inventive hemostatic compositions were prepared as follows by using, *inter alia,* the spray drying method. One part of ORC fiber was combined with 1 part, or 2 parts, or 5 parts, or 0.5 parts of fibrinogen or fibrin sealant (a blend of fibrinogen and thrombin) powders, by weight of the fibrinogen. Thus, as an example, 10 g of ORC powder was combined with 5 g fibrinogen in the fibrin sealant mixed powders.

Spray drying is a drying method used to create powder from a solution, suspension or emulsion that is atomized through a spray nozzle in a hot airflow and dried instantly. Spray drying process is controlled by several process parameters, among them are: column air flow and temperatures, nozzle size and atomizing air flow rate, material flow rate, etc.

In exemplary procedures, thrombin and fibrinogen powders were produced using (each one separately) a spray drying method (Spray dryer: 4M8-TriX spray dryer was used (by ProCepT NV, Zelzate, Belgium)) as follows.

Prepared thrombin and fibrinogen porcine fibrinogen solutions were drawn into a syringe and placed inside the syringe pump of the spray dryer at various amounts. The syringe pump was set to the desired flow rate with feed valve closed. While the syringe pump feed valve was closed, the spray dryer was activated and the desired atomizing gas flow rate, drying gas flow rate, drying gas temperature, cooling gas flow rate, and cyclone gas flow rate were set.

The cooling gas flow rate and temperature were selected such as not to disrupt laminar flow in the drying column, but to reduce the gas flow temperature to below the glass transition temperature of the composition in order to prevent powder from sticking to the glass parts.

The spray dryer was allowed to run until a steady state was reached where the actually measured value of the parameters reached the set levels and remained steady. The feed valve was then opened, allowing the thrombin/fibrinogen solutions to flow through the feed inlet to the spray nozzle to be atomized by the atomizing gas flow to small droplets which were then dried in the drying column. Spray dried powder was formed, and was then collected in the powder outlet of the cyclone of the spray dryer.

The spray dried powder recovered from the cyclone was weighed in a de-humidifier at a relative humidity of below 30 % and divided into samples of between 100 - 200 mg. Each sample was individually sealed in a test tube with a plug and sealed with Parafilm^{®} (Bemis, Oshkosh, Wisconsin, USA) until evaluation.

Parameters for spraying: fan pressure 0.3 bar; atomizing pressure 3 bar; flow rate 130 ml/min; liquid pressure 16 kpa; Nozzle Diameter 0.7 mm; agitation speed 90 rpm/min.

In exemplary procedures, the powders were further granulated using high shear process.

*Equipment details:* Balance: SS1000; High shear mixer: Mini-CG 1L; Syringe pump: LSP01-1C; Vacuum dry: Vacucell 111; Vibrating screen: Fritsch analysette 3.

*Environment:* Temperature: 22-26 °C; Relative Humidity: 50 %-70 %.

In exemplary procedures, the granulated powder was created in a high shear mixing/shearing reactor, having a 150-500 rpm speed mixing blade and 150-1000 rpm cutting blade. Specifically, in the premixing step, the materials were weighed for a total weight of 100 g. The materials were put into the 1L bowl of high shear mixer. Chopper speed was set at 100 rpm, impeller speed was set at 200 rpm, mixing for 5 mins. Next, in the granulation step, the chopper speed was set at 300 rpm, impeller speed was set at 1000 rpm, and the sealing pressure was set at 0.05 MPa. Using a syringe pump, 8-16 ml glycerol solution was sprayed into the bowl to bind the particles, in a flowrate of 4 ml/min, and nozzle size 0.4 mm. Atomization pressure was 0.05 MPa. Granulation time: 2-4 mins.

Next, in the post-granulation step, the chopper speed was adjusted to 300 rpm, and the impeller speed to 1000 rpm. Granulation was then continued for 40-120 s. It is to note that since glycerol-water was used as a binding agent during the process, no liquid was visible.

Next, a first vacuum drying was applied: the obtained composition was transferred to a tray and put into vacuum drying box. Vacuum pressure was set to approximately 0 Pa for 0.5-1 hours. Next, the powder was sieved: the powder was transferred to the vibrating screen tray, the sieve size was in proper order 500/355 micron. The amplitude was set at 1.5 mm, sieving time: 10 mins. Next, a second vacuum drying was applied: the powder was transferred into the vacuum drying box, kept for drying for 3 hours, with the vacuum pressure being approximately 0 Pa. Finally, the product was collected.

Materials in the formulation: 20-80 % fibrinogen powder; 2000-4000 IU/dose thrombin powder; 10-50 % ORC powder; 2-4 % CaCl₂ powder; 0-25 % lysine powder, by weight.

In exemplary preliminary procedures, 1.2 to 1.5 g of tested powder samples was combined with 4 to 5 ml of dispersant comprising saline and glycerol at certain ratios (v/v): 15 % glycerol, 30 % glycerol, 40 % glycerol, 50 % glycerol, and 70 % glycerol. As comparative samples, the following samples were further tested: 1:1 ORC to fibrinogen without thrombin; 2:1 ORC to BSA without thrombin (ratios are by weight). The details of the tested samples are provided below in Example 2 below.

### EXAMPLE 2: CHARACTERIZATION OF THE FLOWABILITY PROPERTY OF SELECTED SAMPLES - PRELIMINARY TESTS

In exemplary procedures, experiments were carried out to test various samples with respect to their pasty consistency.

*Preliminary Trial:* in exemplary procedures, an amount of 1.2 g of tested powder samples was dispersed in 4 ml dispersant comprising saline and 50 % glycerol (v/v). The tested samples were: (i) the above-mentioned powder having ORC-to-fibrinogen weight ratio of 2:1 (with thrombin, 3300 IU per 1.2 g); (ii) the above-mentioned powder having ORC-to-fibrinogen weight ratio of 2:1 but without thrombin; (iii) a powder comprising ORC and BSA with ORC-to-BSA weight ratio of 2:1, with thrombin; (iv) ORC; and (v) a powder comprising ORC and thrombin (without fibrinogen).

The results are shown in **Figure 1** (pictures were taken shortly after incorporating the blend in the dispersant). The results show that only sample (i) provided a pasty composition upon the dispersion in the above-mentioned dispersant. The other samples exhibited flowability which was higher, resulted in composition not being capable of staying at a wound site, and in a slower gelation rate.

*Determining the Amount of the Dispersant:* in exemplary procedures, a dispersant comprising saline and 50 % glycerol (v/v) was chosen, and a powder having ORC-to-fibrinogen weight ratio of 1:1 and 2:1 (with or without thrombin; 3300 IU per dose, i.e. per 1.2 g). The samples further comprised calcium and lysine as detailed in Example 1 above. The samples were evaluated in respect to their flowability with the aim of providing a flowable composition being not too thick (which is hard to push out) on the one hand, and on the other hand, not too thin. The tested samples are summarized in Table 1 below.

**Table 1 Tested Samples for Determining the Powder to Dispersant Ratio (w/v)**

| **Sample No.** | **Powder: ORC-to-fibrinogen Weight Ratio, w/wo Thrombin** | **Powder (g) + Dispersant (ml)** |
|---|---|---|
| 1 | 2:1 | 1.5 + 3 |
| 2 | 2:1 | 1.5 + 4 |
| 3 | 2:1 | 1.5 + 5 |
| 4 | 2:1 + thrombin | 1.5 + 3 |
| 5 | 2:1 + thrombin | 1.5 + 4 |
| 6 | 2:1 + thrombin | 1.5 + 5 |
| 7 | 1:1 | 1.2 + 3 |
| 8 | 1:1 | 1.2 + 4 |
| 9 | 1:1 | 1.2 + 5 |
| 10 | 1:1 | 1.5 + 5 |
| 11 | 1:1+ thrombin | 1.5 +3 |
| 12 | 1:1+ thrombin | 1.5 + 4 |
| 13 | 1:1+ thrombin | 1.5 + 5 |
| 14 | 1:1+ thrombin | 1.5 + 5 |

It can be seen that 3 ml dispersant provided too thick composition, and with 5 ml the composition becomes very thin. The results are visualized in **Figure 2****.** In view of these results, the tested samples of the following Examples comprise 1.2 g of powder dispersed in 4 ml dispersant.

*Flowability and Clotting Time of Tested Samples with 1:2 to 1:5 Ratios with 70 % to 80 % Glycerol:* additional exemplary tests were performed for the ORC-to-fibrinogen weight ratios, 2:1, 1:1, 1:2, and 1:5. In exemplary procedures, 1.2 g of tested samples was dispersed in 4 ml dispersant comprising saline and 70 % or 80 % glycerol (v/v). The samples further comprised thrombin, calcium and lysine as detailed in Example 1 above.

The results are shown in **Figure 3** (pictures were taken shortly after incorporating the blend in the dispersant) for the *in vitro* porcine blood clotting time. The results show that the 70 % glycerol-in-dispersant samples provided a pasty composition upon the dispersion in the above-mentioned dispersant, whereas 80 % glycerol samples exhibited higher flowability (mainly for the 2:1 to 1:2 samples), resulted in composition less capable of staying at a wound site, and also in a slower clotting rate (above 180 s for the 1:5 sample; the blood clotting time for the 70 % glycerol samples, 1:2 and 1:5 ratios, was 70 sec). It can be concluded that about 70 % glycerol is the upper concentration limit for the herein disclosed compositions.

### EXAMPLE 3: RHEOLOGICAL PROPERTIES

In exemplary procedures, the samples were tested with respect to their viscosity.

An amount of 1.2 g of tested powder samples was dispersed in 4 ml dispersant comprising saline and 50 % glycerol (v/v) having ORC-to-fibrinogen weight ratio of 1:5 to 2:1. The samples further comprise thrombin (3300 IU per 1.2 g calcium and lysine as detailed in Example 1 above).

*Viscosity:* mixed sample was sandwiched between a 25-mm parallel plate and the Peltier plate surface. The gap between the two plates was set to 1.00 mm for all measurements. Rotation mode test with the shear rate being set to 0.5 1/s and temperature of 25 °C were used for all measurements.

The viscosity was determined by the ratio of shear stress to shear rate. The results are presented in **Figure 4** showing that the at the weight ratio of ORC-to-fibrinogen ranges from 1:5 to 2:1 the viscosity is greater than about 400 and is less than about 3000 Pa·s.

*Amplitude Sweep Test:* in an exemplary set of experiments, shear loss (G') and shear storage (G") modulus of the samples were evaluated *via* shear rheometer. The measurements were conducted using Anton Paar Modular Compact Rheometer MCR102. All tests were conducted at a temperature of 25 °C. In a strain sweep test, the frequency of the test is fixed and the amplitude is incrementally increased. A strain sweep tests were conducted to determine the linear viscoelastic regime of the samples. Sweep tests were then performed at a shear strain (oscillating) range of 0.01 to 100 %, angular frequency in the range of up to 10 rad/sec. The amplitude is the maximum of the oscillatory motion. For the analysis the storage modulus G' and the loss modulus G" are plotted against the deformation. If G' > G", then the sample shows a gel-like or solid structure and can be termed a viscoelastic material. The limit of the linear viscoelastic region is also first determined. In the linear viscoelastic region, the functions of G' and G" show constant plateau values indicating the range in which the test can be carried out without destroying the structure of the sample. The intersection of G' and G" is the yield point (τ). τ = F/A with shear stress τ (tau), shear force F (in N) and shear area A (in m²).

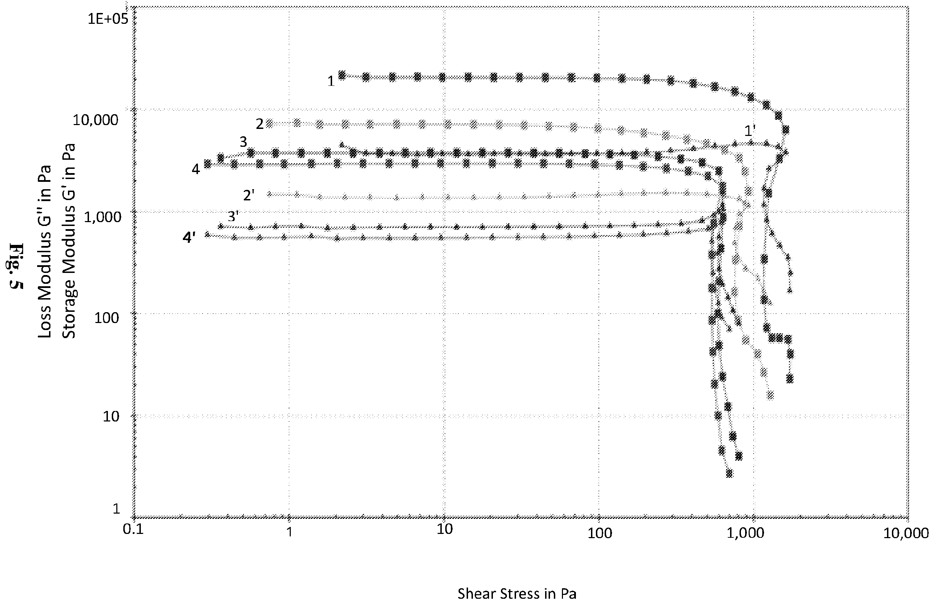

The results presented in **Figure 5** show the linear viscoelastic regions, with the storage modulus (G') and loss modulus (G") remaining unchanged, meaning that the samples with weight ratios of ORC-to-fibrinogen of from 1:5 to 2:1 have strong stability. The results also show that tau value for the 2:1 sample has highest yield point, having a longer linear viscoelastic region, and the 1:5 sample has the shortest linear viscoelastic region, meaning that the ORC-to-fibrinogen ratio of 1:5 may be less preferable.

### EXAMPLE 4: CLOTTING AND STABILITY TESTS

*Ex-vivo Clotting of Porcine Blood: Ex-vivo* clotting of blood by several inventive and comparative compositions was tested as follows. An amount of 1.2 g of each sample was dissolved in 4 ml saline + glycerol (v/v), for 20 to 40 mins, forming a paste. An amount of 2 ml of blood were taken into a penicillin bottle, and 1 ml (about 0.24 g powder or 0.5 g SURGIFLO^{®} gelatin matrix) sample was added followed by and gentle shaking for three times with a vortex meter (IKA VORTEX 3). The coagulation time was observed and recorded as well as the state of each sample at different time points. The time for clotting formation was evaluated by tilting the vials overtime. Samples were tested after sample preparation (reconstitution then mixing the powder with dispersant back and forth several times to form paste) upon 0.5 h, 2 h, and 20 h. The clotting time was determined by visual observation of blood flow through inverted sample vials. At the end point of the clotting time the blood clot does not come off the vial. The results are summarized in Table 2 below.

**Table 2 Blood Clotting Time of Various Samples**

| **Sample** | **Time(s)** | | |
|---|---|---|---|
| | **0.5h** | **2h** | **20h** |
| **(1)** 2:1 50V% | 55 | 22 | 20 |
| **(2)** 2:1 40V% | 18 | 15 | 14 |
| **(3)** 2:1 30V% | 70 | 14 | 28 |
| **(4)** 2:1 15V% | 180 | 60 | 15 |
| **(5)** 1:1 50V% | 33 | 13 | 14 |
| **(6)** 1:1 40V% | 50 | 20 | 13 |
| **(7)** 1:1 30V% | 50 | 12 | 22 |
| **(8)** 1:1 15V% | 15 | 16 | 11 |

The results show that for the ORC-to-fibrinogen weight ratios of 2:1 to 1:1, the preferred concentration of glycerol in the dispersant (without compromising the enzyme activity, as described below) is above 15 % to less than 50 %, by volume, providing a clotting time of less than 60 sec for each time point.

*Effect of pH and Glycerol Concentration on Stability:* additional experiments were carried out to test various samples after reconstitution in various pH values with respect to their stability, using L-lysine to adjust pH. Samples tested: (1) ORC-to-fibrinogen 2:1, saline + 50 % glycerol, pH 4.48 ("2:1, 50 %, 4.48"); and accordingly: (2) 2:1, 50 %, 5.38; (3) 2:1, 50 %, 6.53; (4) 2:1, 40 %, 4.44; (5) 2:1, 30 %, 4.44; (6) 2:1, 15 %, 4.33; (7) 1:1, 50 %, 5.03; (8) 1:1, 50 %, 5.46; (9) 1:1, 40 %, 5.05; (10) 1:1, 30 %. 4.81; (11) 1:1, 15 %, 4.82; (12) SURGIFLO^{®} + thrombin. The samples further comprise thrombin, calcium and lysine as detailed in Example 1 above. The results presented in **Figure 6** show a liquid phase separation for the 15 % glycerol sample after 20 h.

*Effect of pH on the Clotting Time:* in exemplary procedures, the pH effect on the clotting time was further tested with 2:1 and 1:1 formulations (50 % glycerol (v/v), saline solution). The protocol for testing thrombin activity is detailed above, and was carried out at room temperature. The results are presented in **Table 3** below and in **Figure 7****.**

**Table 3 Blood Clotting Time at Various pH Values**

| **Blood Clotting Time** | | | |
|---|---|---|---|
| **Sample** | **0.5h** | **2h** | **20h** |
| 2:1 pH=4.48 | 55 | 22 | 20 |
| 2:1 pH=5.38 | 75 | 12 | 30 |
| 2:1 pH=6.53 | 70 | 60 | 140 |
| 1:1 pH=5.03 | 33 | 13 | 14 |
| 1:1 pH=5.46 | 50 | 23 | 15 |
| 50% (V/V) Glycerol/Saline 1.2g+4ml | | | |

The results further confirm that the preferred pH range is about 4.5 to 6.5, as in this range the blood clotting time is not higher than 50 sec at at-least two tested times.

### EXAMPLE 5: ACTIVITY TESTS OVER TIME

*Tested Samples for the 2:1 to 1:1 Ratios:* the thrombin activity in the tested sample is represented by the specific activity reported as IU/mg. Tested sample was hydrated in alkaline carbonate to limit thrombin activity and minimize fibrin formation. A small volume of the sample dissolved in the carbonate solution was neutralized with a large volume of buffered fibrinogen solution to allow the clotting reaction. In exemplary procedures, 1.2 g of tested samples was dispersed in 4 ml dispersant containing various concentrations of glycerol (% v/v) in the saline solution and pH values. Table 4 presents the result of each tested sample having certain ORC-to-fibrinogen weight ratios, 2:1 or 1:1, and glycerol concentrations (% v/v) with respect of the thrombin (enzyme) activity (IU per 1.2 g) after 0.5 h, 1.5 h, 2.5 h, 4.5 h, 20 h according to the following protocol.

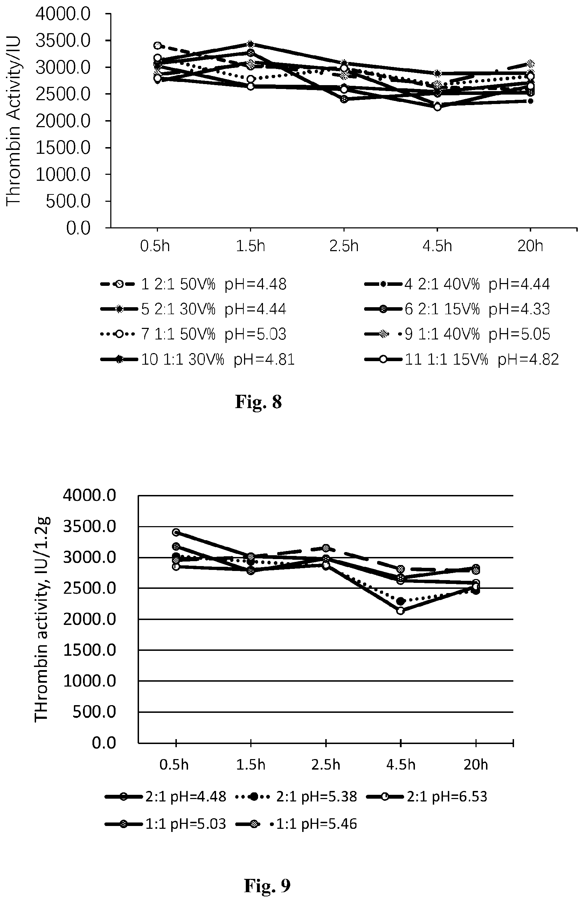

The results are further shown in **Figure 8****.**

**Table 4 Thrombin Activity at Different Concentrations of Glycerol (the samples further comprise thrombin, calcium and lysine as detailed in Example 1 above)**

| **Sample** | **pH** | **Activity IU per 1.2 g** | | | | |
|---|---|---|---|---|---|---|
| | | **0.5 h** | **1.5 h** | **2.5 h** | **4.5 h** | **20 h** |
| **(1)** 2:1 50V% | 4.48 | 3403.9 | 3017.0 | 2976.4 | 2623.9 | 2587.0 |
| **(2)** 2:1 40V% | 4.44 | 2735.5 | 3095.6 | 2949.0 | 2301.6 | 2366.4 |
| **(3)** 2:1 30V% | 4.44 | 3115.1 | 3431.9 | 3071.7 | 2882.9 | 2887.5 |
| **(4)** 2:1 15V% | 4.33 | 3068.6 | 3268.6 | 2402.8 | 2511.3 | 2527.2 |
| **(5)** 1:1 50V% | 5.03 | 3177.0 | 2778.3 | 2980.2 | 2669.5 | 2829.5 |
| **(6)** 1:1 40V% | 5.05 | 2862.5 | 3056.8 | 2840.3 | 2683.5 | 3065.7 |
| **(7)** 1:1 30V% | 4.81 | 3019.1 | 2645.6 | 2627.6 | 2538.6 | 2709.7 |
| **(8)** 1:1 15V% | 4.82 | 2794.5 | 2639.4 | 2585.3 | 2256.7 | 2647.6 |

*pH Effect on Thrombin Stability over Time:* in exemplary procedures, the pH effect on thrombin stability over time was tested with 2:1 and 1:1 formulations (50 % Glycerol (v/v), saline solution). The pH was adjusted using L-lysine powder, and the pH was measured using pH meter with surface electrode (METTLER TOLEDO). The protocol for testing thrombin activity is detailed above and was carried out at room temperature. The results are presented in **Table 5** below and in **Figure 9****.**

**Table 5 pH Effect on Thrombin Stability (the samples further comprise thrombin, calcium and lysine as detailed in Example 1 above)**

| **Enzyme activity (IU/mg)** | | | | | |
|---|---|---|---|---|---|
| **Sample** | **0.5h** | **1.5h** | **2.5h** | **4.5h** | **20h** |
| 2:1 pH=4.48 | 3403.9 | 3017.0 | 2976.4 | 2623.9 | 2587.0 |
| 2:1 pH=5.38 | 3018.9 | 2933.7 | 2855.2 | 2291.4 | 2460.2 |
| 2:1 pH=6.53 | 2851.0 | 2799.9 | 2876.6 | 2136.5 | 2527.2 |
| 1:1 pH=5.03 | 3177.0 | 2778.3 | 2980.2 | 2669.5 | 2829.5 |
| 1:1 pH=5.46 | 2952.8 | 3005.8 | 3151.4 | 2813.9 | 2782.3 |
| 50 % (V/V) Glycerol/Saline 1.2 g + 4 ml | | | | | |

Taken together, the results show that the activity of thrombin did not decrease significantly within 20 h under different formulations and pH conditions, so the formulations showed good stability within 20 h. Nonetheless, the preferred pH range is about 4.5 to 6.5, as within this range, the thrombin activity did not decrease appreciably within 20 hours and kept stable.

### EXAMPLE 6: EFFECT ON THICKNESS

In exemplary procedures, a certain amount of various samples was taken and stacked on the test platform of a thickness tester (CDK). The thickness of the sample was let to drop naturally, with the sample being compressed. The thickness of the sample was then recorded, observing the change of the thickness of the sample over time. Various samples having certain ORC-to-fibrinogen weight ratios, 2:1 or 1:1, and glycerol concentrations (%v/v; 4 ml per 1.2 g) were tested, and the results are presented in Table 6. The samples further comprised thrombin, calcium and lysine as detailed in Example 1 above.

**Table 6 Different Formulations Effect on Thickness over Time**

| **Thickness (mm)** | | | | | |
|---|---|---|---|---|---|
| **pH** | **Sample** | **0 h** | **2 h** | **5.5 h** | **24 h** |
| 4.97 | 2:1 50% | 0.25 | 0.22 | 0.24 | 0.28 |
| 4.99 | 2:1 30% | 0.07 | 0.20 | 0.22 | 0.29 |
| 5.1 | 1:1 50% | 0.21 | 0.28 | 0.29 | 0.22 |
| 4.87 | 1:1 30% | 0.13 | 0.26 | 0.28 | 0.36 |
| 4.85 | 1:2 50% | 0.28 | 0.44 | 0.57 | 0.62 |
| 4.77 | 1:2 30% | 0.65 | 1.21 | 1.13 | 1.28 |
| 5.47 | 1:5 50% | 0.37 | 0.49 | 0.80 | 0.91 |
| 5.43 | 1:5 30% | 0.87 | 0.72 | 1.03 | 0.96 |

It can be concluded that with ORC-to-fibrinogen ratio below 2:1 (1:1, 1:2, and 1:5), the reconstituted paste becomes more rigid, having a higher thickness, with the 1:5 ratio becoming too rigid. **Figure 10** presenting the results of representative tested samples further illustrates this feature.

### EXAMPLE 7: IN VIVO EVALUATION IN PORCINE LIVER AND SPLEEN MODELS

In exemplary procedures, selected samples were tested to evaluate the hemostatic efficacy of flowable hemostasis compositions using a swine punch bleeding models (on spleen and liver biopsy punch bleeding models) under heparinized condition.

In exemplary procedures, the samples presented in Table 8 were tested (in 50 % v/v glycerol/saline).

In exemplary procedures, a punch hole of 5 mm was made deep on the surface of the spleen or liver with a 6 mm puncher. Prior to testing for article application, excess blood was blotted/removed from the target bleeding site with gauze or by using suction so the hemostatic pastes could be applied directly to the bleeding site with the bleeding surface. A sufficient amount of paste was applied to the site to cover the entire bleeding area with multiple layers.

A tamponade was initially maintained for a 3-minute period, then the gauze was lifted from the site and the site was observed for up to 60 seconds. When free flowing blood was observed at any time during the observation period, tamponade was again applied over the gauze and held for an additional minute. Hemostasis success or failure was determined at press sample for 3-, 5- and 10-minutes post application. The trial was aborted and results were recorded in the raw data as greater than 10 minutes. Once bleeding had been ceased, an excessive sample was irrigated away with 10 mL of saline.

*Acceptance Criteria:* hemostatic efficacy was determined by the cessation of free flow bleeding at 1-, 2-, 3- and 4- minutes post-application using a 0-5 rating scale (Table 7 and **Figure 11**) to measure the bleeding.

Hemostasis was considered successful if: "No bleeding- 0" or "ooze- 1" post article application was achieved.

**Table 7 Bleeding Scores - Criteria**

| **Bleeding Score** | **Degree of Bleeding** | |
|---|---|---|
| **0** | No bleeding at wound site | No bleeding |
| **1** | Blood oozing at wound site | Very minor capillary, arteriole, or venule oozing |
| **2** | Very mild bleeding | Capillary, arteriole, or venule oozing |
| **3** | Mild bleeding | Flowing capillary, arteriole, or venule oozing |
| **4** | Moderate bleeding | Flowing venous and/or arterial bleeding |
| **5** | Sever bleeding | Pulsatile or spurting arterial or high-volume venous bleeding |

The results are summarized in Table 8.

**Table 8 Hemostatic Effectiveness of Selected Samples. The samples further comprise thrombin, calcium and lysine as detailed in Example 1 above.**

| **Test Sample** | **Bleeding Model and Bleeding Score Rating** | | **Bleeding Score Post** Application | **Accumulated Tamponade Time** |
|---|---|---|---|---|
| SURGIFLO^{®} + Thrombin | Liver Punch Model | 4 | 2 | 4 min |
| | | 3 | 0 | 3 min |
| | | 5 | 2 | 4 min |
| | | 4 | 1 | 2 min |
| | Spleen Punch Model | 3 | 1 | 2 min |
| | | 4 | 3 | 3 min |
| | | 4 | 2 | 3 min |
| **Hemostatic Effectiveness** | | | **42.9% (3/7)** | |
| 2:1 of ORC to FIB (THR ~500IU/ml) | Liver Punch Model | 4 | 0 | 3 min |
| | | 5 | 0 | 3 min |
| | | 3 | 1 | 3 min |
| | Spleen Punch Model | 3 | 2 | 4 min |
| 2:1 of ORC to FIB (THR ~1000IU/ml) | Liver Punch Model | 4 | 1 | 4 min |
| | | 4 | 2 | 3 min |
| | | 3 | 0 | 3 min |
| | Spleen Punch Model | 4 | 2 | 2 min |
| | | 4 | 1 | 3 min |
| **Hemostatic effectiveness** | | | **66.7% (6/9)** | |
| 1:1 of ORC to FIB (THR ~500IU/ml) | Liver Punch Model | 4 | 0 | 2 min |
| | | 4 | 1 | 2 min |
| | | 5 | 2 | 2 min |
| | Spleen Punch Model | 3 | 0 | 3 min |
| 1:1 of ORC to FIB (THR ~1000IU/ml) | Liver Punch Model | 4 | 0 | 4 min |
| | Spleen Punch Model | 5 | 1 | 4 min |
| **Hemostatic effectiveness** | | | **83.3% (5/6)** | |

Additional results are presented in **Figure 12****.**

In another set of *in vivo* experiments canine punch model test with different formulation (n=5) was carried out in a similar protocol as in liver and. Canine received an initial loading intravenous bolus of 100-300 IU/kg of heparin sodium. The activated clotting time (ACT) was measured approximately ten minutes after injection. The animal was considered heparinized when ACT is > x3 baseline reading. The results are summarized in **Figure 13****.**

Taken together, the above results illustrate the high hemostatic success rate (above 60%) of the tested samples, with the 1:1 ratio being somewhat superior.

## Claims

1. A two-component flowable composition comprising: (i) a powder comprising oxidized cellulose, fibrinogen and thrombin, having a weight ratio of oxidized cellulose to fibrinogen ranging from about 1:5 to about 2:1, respectively; and (ii) a dispersant comprising glycerol and an aqueous solution, the composition being in the form of a pasty suspension at a room temperature.

2. The composition of claim 1, wherein:
(i) the weight ratio of the OC to fibrinogen ranges from 1:2 to 2:1;
(ii) said OC comprises oxidized regenerated cellulose (ORC); and/or
(iii) at least part of the powder is in an aggregated form.

3. The composition of claim 1 or 2, wherein the glycerol is present at a concentration of (i) above about 15% by volume of said dispersant,
such as above about 40% by volume of said dispersant; and/or
(ii) up to about 70% by volume of said dispersant.

4. The composition of any one of claims 1 to 3:
(i) wherein the dispersant is present at a dispersant-to-powder ratio ranging from 3:1 to 4:1 (v/w; ml per g), respectively;
(ii) wherein the OC is in the milled form;
(iii) further comprising fibrin, optionally wherein the fibrin is at least partially cross-linked; and/or
(iv) wherein the thrombin is present at an amount of about 2000 to about 4000 IU per 1 gr powder.

5. The composition of any one of claims 1 to 4, further comprising:
(i) a calcium salt; and/or
(ii) a buffering agent, optionally wherein the buffering agent is in the form of a powder, and/or optionally wherein the buffering agent is selected from Tris, lysine, or a combination thereof.

6. The composition of any one of claims 1 to 5, wherein the particle size of at least 90% of the powder ranges from 10 to 2,000 µm, and/or wherein the particle size ranges from about 200 to about 900 µm.

7. The composition of any one of claims 1 to 6, being stable for at least about 20 h at pH of 4 to 6.5 at a room temperature.

8. The composition of any one of claims 1 to 7, wherein:
(i) the aqueous solution comprises saline;
(ii) the powder is spray dried and/or high sheared mixed; and/or
(iii) the fibrinogen is present at a concentration range of 60 % to 95 %, or 70 % to 90 %, by weight of the powder.

9. The composition of any one of claims 1 to 8, for use as a hemostat in a bleeding site of a tissue.

10. The composition of any one of claims 1 to 9 for use in a method for treating a bleeding tissue, the method comprising applying said composition to the bleeding tissue.

11. The composition for use according to claim 10, wherein the method comprises minimal invasive surgery e.g., laparoscopy.

12. A method of making the composition of any one of claims 1 to 9, the method comprising combining OC fibers with fibrinogen powder and thrombin powder in conditions allowing forming an aggregated form of said OC fibers, fibrinogen and thrombin, and combining said aggregate with a dispersant comprising glycerol and an aqueous solution.

13. The method of claim 12, wherein the conditions allow at least partial conversion of fibrinogen into fibrin, optionally wherein the conditions allow at least partial crosslinking of the fibrin.

14. The method of claim 12 or 13, wherein the conditions are selected from one or more from (i) to (iii): (i) a temperature in the range of from 0-30°C, (ii) pH of the dispersant being in the range of from 4.5 to 6.5, and (iii) a dispersant being at a dispersant-to-powder ratio ranging from 3:1 to 4:1 (v/w; ml/g), respectively.

15. A kit comprising:
(a) a container containing a powder comprising oxidized cellulose, fibrinogen and thrombin, having a weight ratio of oxidized cellulose to fibrinogen ranging from about 1:5 to about 2:1, respectively; and
(b) a container containing a dispersant comprising glycerol and optionally an aqueous solution, and optionally
(c) instructions for use.

## Patentansprüche

1. Fließfähige Zweikomponentenzusammensetzung, umfassend: (i) ein Pulver, das oxidierte Zellulose, Fibrinogen und Thrombin umfasst, mit einem Gewichtsverhältnis von oxidierter Zellulose zu Fibrinogen jeweils im Bereich von etwa 1:5 bis etwa 2:1; und (ii) ein Dispergiermittel, das Glycerin und eine wässrige Lösung umfasst, wobei die Zusammensetzung bei Raumtemperatur in Form einer pastösen Suspension vorliegt.

2. Zusammensetzung nach Anspruch 1, wobei:
(i) das Gewichtsverhältnis der OC zu Fibrinogen im Bereich von 1:2 bis 2:1 liegt;
(ii) die OC oxidierte regenerierte Zellulose (ORC) umfasst; und/oder
(iii) mindestens ein Teil des Pulvers in aggregierter Form vorliegt.

3. Zusammensetzung nach Anspruch 1 oder 2, wobei das Glycerin in einer Konzentration von (i) über etwa 15 Vol.-% des Dispergiermittels,
wie über 40 Vol.-% des Dispergiermittels; und/oder
(ii) bis zu etwa 70 Vol.-% des Dispergiermittels vorhanden ist.

4. Zusammensetzung nach einem der Ansprüche 1 bis 3:
(i) wobei das Dispergiermittel in einem Dispergiermittel-zu-Pulver-Verhältnis im Bereich von 3:1 bis 4:1 (v/w; bzw. ml pro g) vorhanden ist;
(ii) wobei die OC in gemahlener Form vorliegt;
(iii) ferner umfassend Fibrin, wobei das Fibrin optional mindestens teilweise vernetzt ist; und/oder
(iv) wobei das Thrombin in einer Menge von etwa 2000 bis etwa 4000 IE pro 1 g Pulver vorhanden ist.

5. Zusammensetzung nach einem der Ansprüche 1 bis 4, ferner umfassend:
(i) ein Calciumsalz; und/oder
(ii) ein Puffermittel, wobei das Puffermittel optional in Pulverform vorliegt und/oder wobei das Puffermittel optional aus Tris, Lysin oder einer Kombination davon ausgewählt ist.

6. Zusammensetzung nach einem der Ansprüche 1 bis 5, wobei die Partikelgröße von mindestens 90 % des Pulvers im Bereich von 10 bis 2.000 µm liegt und/oder wobei die Partikelgröße im Bereich von etwa 200 bis etwa 900 µm liegt.

7. Zusammensetzung nach einem der Ansprüche 1 bis 6, die bei einem pH-Wert von 4 bis 6,5 bei Raumtemperatur mindestens etwa 20 h stabil ist.

8. Zusammensetzung nach einem der Ansprüche 1 bis 7, wobei:
(i) die wässrige Lösung Kochsalzlösung umfasst;
(ii) das Pulver sprühgetrocknet und/oder unter hoher Scherung gemischt ist; und/oder
(iii) das Fibrinogen in einer Konzentration im Bereich von 60 Gew.-% bis 95 Gew.-% oder 70 Gew.-% bis 90 Gew.-% des Pulvers vorliegt.

9. Zusammensetzung nach einem der Ansprüche 1 bis 8 zur Verwendung als Hämostat an einer blutenden Gewebestelle.

10. Zusammensetzung nach einem der Ansprüche 1 bis 9 zur Verwendung in einem Verfahren zum Behandeln eines blutenden Gewebes, wobei das Verfahren das Auftragen der Zusammensetzung auf das blutende Gewebe umfasst.

11. Zusammensetzung zur Verwendung nach Anspruch 10, wobei das Verfahren eine minimalinvasive Operation, z. B. Laparoskopie, umfasst.

12. Verfahren zum Herstellen der Zusammensetzung nach einem der Ansprüche 1 bis 9, wobei das Verfahren das Kombinieren von OC-Fasern mit Fibrinogenpulver und Thrombinpulver unter Bedingungen umfasst, die das Bilden einer aggregierten Form der OC-Fasern, des Fibrinogens und des Thrombins, und das Kombinieren des Aggregats mit einem Dispergiermittel, das Glycerin und eine wässrige Lösung umfasst, ermöglichen.

13. Verfahren nach Anspruch 12, wobei die Bedingungen eine mindestens teilweise Umwandlung von Fibrinogen in Fibrin ermöglichen, wobei die Bedingungen optional eine mindestens teilweise Vernetzung des Fibrins ermöglichen.

14. Verfahren nach Anspruch 12 oder 13, wobei die Bedingungen aus einem oder mehreren von (i) bis (iii) ausgewählt sind: (i) einer Temperatur im Bereich von 0-30 °C, (ii) einem pH-Wert des Dispergiermittels im Bereich von 4,5 bis 6,5 und (iii) einem Dispergiermittel mit einem Dispergiermittel-zu-Pulver-Verhältnis im Bereich von 3:1 bis 4:1 (v/w; bzw. ml/g).

15. Kit, umfassend:
(a) einen Behälter, der ein Pulver enthält, das oxidierte Zellulose, Fibrinogen und Thrombin umfasst, mit einem Gewichtsverhältnis von oxidierter Zellulose zu Fibrinogen jeweils im Bereich von etwa 1:5 bis etwa 2:1; und
(b) einen Behälter, der ein Dispergiermittel enthält, das Glycerin und optional eine wässrige Lösung umfasst, und optional
(c) Gebrauchsanweisungen.

## Revendications

1. Composition fluide à deux composants comprenant : (i) une poudre comprenant de la cellulose oxydée, du fibrinogène et de la thrombine, ayant un rapport pondéral de la cellulose oxydée au fibrinogène allant d'environ 1:5 à environ 2:1, respectivement ; et (ii) un dispersant comprenant du glycérol et une solution aqueuse, la composition étant sous la forme d'une suspension pâteuse à une température ambiante.

2. Composition selon la revendication 1, dans laquelle :
(i) le rapport pondéral de la CO au fibrinogène va de 1:2 à 2:1 ;
(ii) ladite CO comprend de la cellulose régénérée oxydée (CRO) ; et/ou
(iii) au moins une partie de la poudre est sous une forme agrégée.

3. Composition selon la revendication 1 ou 2, dans laquelle le glycérol est présent à une concentration (i) supérieure à environ 15 % en volume dudit dispersant,
telle que supérieure à environ 40 % en volume dudit dispersant ; et/ou
(ii) allant jusqu'à environ 70 % en volume dudit dispersant.

4. Composition selon l'une quelconque des revendications 1 à 3 :
(i) dans laquelle le dispersant est présent à un rapport du dispersant à la poudre allant de 3:1 à 4:1 (volume/poids ; ml par g), respectivement ;
(ii) dans laquelle la CO est sous la forme broyée ;
(iii) comprenant en outre de la fibrine, facultativement dans laquelle la fibrine est au moins partiellement réticulée ; et/ou
(iv) dans laquelle la thrombine est présente en une quantité d'environ 2000 à environ 4000 IU pour 1 g de poudre.

5. Composition selon l'une quelconque des revendications 1 à 4, comprenant en outre :
(i) un sel de calcium ; et/ou
(ii) un agent tampon, facultativement dans laquelle l'agent tampon est sous la forme d'une poudre, et/ou facultativement dans laquelle l'agent tampon est choisi parmi Tris, lysine, ou une combinaison de ceux-ci.

6. Composition selon l'une quelconque des revendications 1 à 5, dans laquelle la taille de particules d'au moins 90 % de la poudre va de 10 à 2000 µm, et/ou dans laquelle la taille de particules va d'environ 200 à environ 900 µm.

7. Composition selon l'une quelconque des revendications 1 à 6, étant stable pendant au moins environ 20 h à un pH de 4 à 6,5 à une température ambiante.

8. Composition selon l'une quelconque des revendications 1 à 7, dans laquelle :
(i) la solution aqueuse comprend une solution saline ;
(ii) la poudre est séchée par atomisation et/ou mélangée sous cisaillement élevé ; et/ou
(iii) le fibrinogène est présent à une plage de concentration de 60 % à 95 %, ou 70 % à 90 %, en poids de la poudre.

9. Composition selon l'une quelconque des revendications 1 à 8, pour une utilisation en tant qu'hémostatique dans un site de saignement d'un tissu.

10. Composition selon l'une quelconque des revendications 1 à 9 pour utilisation dans un procédé permettant de traiter un tissu qui saigne, le procédé comprenant l'application de ladite composition au tissu qui saigne.

11. Composition pour utilisation selon la revendication 10, dans laquelle le procédé comprend une chirurgie mini-invasive, par exemple une laparoscopie.

12. Procédé de fabrication de la composition selon l'une quelconque des revendications 1 à 9, le procédé comprenant la combinaison de fibres de CO avec de la poudre de fibrinogène et de la poudre de thrombine dans des conditions permettant la formation d'une forme agrégée desdites fibres de CO, dudit fibrinogène et de ladite thrombine, et la combinaison dudit agrégat avec un dispersant comprenant du glycérol et une solution aqueuse.

13. Procédé selon la revendication 12, dans lequel les conditions permettent une conversion au moins partielle de fibrinogène en fibrine, facultativement dans lequel les conditions permettent une réticulation au moins partielle de la fibrine.

14. Procédé selon la revendication 12 ou 13, dans lequel les conditions sont choisies parmi un ou plusieurs parmi (i) à (iii) : (i) une température dans la plage allant de 0 à 30 °C, (ii) un pH du dispersant étant dans la plage allant de 4,5 à 6,5, et (iii) un dispersant étant à un rapport au dispersant à la poudre allant de 3:1 à 4:1 (volume/poids ; ml/g), respectivement.

15. Trousse comprenant :
(a) un récipient contenant une poudre comprenant de la cellulose oxydée, du fibrinogène et de la thrombine, ayant un rapport pondéral de la cellulose oxydée au fibrinogène allant d'environ 1:5 à environ 2:1, respectivement ; et
(b) un récipient contenant un dispersant comprenant du glycérol et facultativement une solution aqueuse, et facultativement
(c) un mode d'emploi.
